# EUROPEAN PATENT SPECIFICATION

(11) **EP 1 242 111 B1**
(45) Date of publication and mention of the grant of the patent: **21.05.2008**
(21) Application number: 00975670.1
(22) Date of filing: 16.11.2000
(51) Int. Cl.: A61K 38/17

(54) **AN ANIMAL MODEL FOR STUDYING HORMONE SIGNALLING AND METHOD OF MODULATING THE SIGNALLING**
TIERMODELL FÜR HORMONSIGNALSTUDIEN UND VERFAHREN ZUM VERÄNDERN DER SIGNALÜBERTRAGUNG
MODELE ANIMAL D'ETUDE DE LA SIGNALISATION HORMONALE ET PROCEDE DE MODULATION DE CETTE SIGNALISATION

(30) Priority: 16.11.1999 AU PQ408299; 29.05.2000 AU PQ781200
(43) Date of publication of application: 25.09.2002
(73) Proprietor: THE WALTER AND ELIZA HALL INSTITUTE OF MEDICAL RESEARCH, Parkville, VIC 3052 (AU)
(72) Inventor: ALEXANDER, Warren, Scott, Moonee Ponds, VIC 3039 (AU); METCALF, Donald, Balwyn, VIC 3103 (AU); GREENHALGH, Christopher, John, North Balwyn, VIC 3104 (AU)
(74) Representative: Jones, Elizabeth Louise
(86) International application number: PCT/AU2000/001398
(87) International publication number: WO 2001/035732

(56) References cited:
- WO-A-00/20624
- WO-A-00/37636
- WO-A-00/63357
- WO-A-96/39427
- WO-A-98/20023
- STARR R ET AL: "A family of cytokine-inducible inhibitors of signalling" NATURE, MACMILLAN JOURNALS LTD. LONDON, GB, vol. 387, 26 June 1997 (1997-06-26), pages 917-921, XP002085491 ISSN: 0028-0836
- HILTON D J ET AL: "Twenty proteins containing a C-terminal SOCS box form five structural classes" PROCEEDINGS OF THE NATIONAL ACADEMY OF SCIENCES OF USA, NATIONAL ACADEMY OF SCIENCE. WASHINGTON, US, vol. 95, 1998, pages 114-119, XP002085497 ISSN: 0027-8424
- BJÖRBAEK C ET AL: "IDENTIFICATION OF SOCS-3 AS A POTENTIAL MEDIATOR OF CENTRAL LEPTIN RESISTANCE" MOLECULAR CELL, CELL PRESS, CAMBRIDGE, MA, US, vol. 1, no. 1, March 1998 (1998-03), pages 619-625, XP002099320 ISSN: 1097-2765
- METCALF D. ET AL.: 'Gigantism in mice lacking suppressor of cytokine signaling-2' LETTERS TO NATURE vol. 405, 29 July 2000, pages 1069 - 1073, XP002958016
- METCALF D.: 'The SOCS-1 story' EXPERIMENTAL HEMATOLOGY vol. 27, pages 1715 - 1723, XP002958017
- STARR R. ET AL.: 'Liver degeneration and lymphoid deficiencies in mice lacking suppressor of cytokine signaling-1' PROC. NATL. ACAD. SCI. USA vol. 95, pages 14395 - 14399, XP002958018
- NAKA T. ET AL.: 'Accelerated apoptosis of lymphocytes by augmented induction of Bax in SSI-1 (STAT-induced STAT inhibitor-1) deficient mice' PROC. NATL. ACAD. SCI. USA vol. 95, pages 15577 - 15582, XP002958019
- ALEXANDER W.S. ET AL.: 'Suppressors of cytokine signaling (SOCS): negative regulators of signal transduction' JOURNAL OF LEUKOCYTE BIOLOGY vol. 66, pages 588 - 592, XP002958020
- FAVRE H. ET AL.: 'Dual effects of suppressor of cytokine signaling (SOCS-2) on growth hormone signal transduction' FEBS LETTERS vol. 453, pages 63 - 66, XP004259803
- DEY B.R. ET AL.: 'Interaction of human suppressor of cytokine signaling (SOCS)-2 with the insulin-like growth factor-1 receptor' J. BIOLOGICAL CHEMISTRY vol. 273, pages 24095 - 24101, XP002958021
- PEZET A. ET AL.: 'Inhibition and restoration of prolactin signal transduction by suppressors of cytokine signaling' J. BIOLOGICAL CHEMISTRY vol. 274, pages 24497 - 24502, XP002958022

### SEQUENCE LISTING

<110> THE WALTER AND ELIZA HALL INSTITUTE OF MEDICAL RES
<120> A METHOD
<130> 2338102/EJH
<140> International
   <141> 2000-11-16
<150> PQ4082
   <151> 1999-11-16
<150> PQ7812
   <151> 2000-05-29
<160> 2
<170> Patent In Ver. 2.1
<210> 1
   <211> 1121
   <212> DNA
   <213> mouse
<220>
   <221> CDS
   <222> (223)..(816)
   SEQ ID No. 1
<400> 1
   gcgatctgtg ggtgacagtg tctgcgagag actttgccac accattctgc cggaatttgg 60
   agaaaaagaa ccagccgctt ccagtcccct ccccctccgc caccatttcg gacaccctgc 120
   acactctcgt tttggggtac cctgtgactt ccaggcagca cgcgaggtcc actggcccca 180
   gctcgggcga ccagctgtct gggacgtgtt gactcatctc cc atg acc ctg cgg    234
                                                   Met Thr Leu Arg
                                                   1
   tgc ctg gag ccc tcc ggg aat gga gcg gac agg acg cgg agc cag tgg   282
   Cys Leu Glu Pro Ser Gly Asn Gly Ala Asp Arg Thr Arg Ser Gln Trp
    5                  10                  15                  20
   ggg acc gcg ggg ttg ccg gag gaa cag tcc ccc gag gcg gcg cgt ctg   330
   Gly Thr Ala Gly Leu Pro Glu Glu Gln Ser Pro Glu Ala Ala Arg Leu
                   25                  30                  35
   gcg aaa gcc ctg cgc gag ctc agt caa aca gga tgg tac tgg gga agt   378
   Ala Lys Ala Leu Arg Glu Leu Ser Gln Thr Gly Trp Tyr Trp Gly Ser
               40                  45                  50
   atg act gtt aat gaa gcc aaa gag aaa tta aaa gag gct cca gaa gga   426
   Met Thr Val Asn Glu Ala Lys Glu Lys Leu Lys Glu Ala Pro Glu Gly
           55                  60                  65
   act ttc ttg att aga gat agt tcg cat tca gac tac cta cta act ata   474
   Thr Phe Leu Ile Arg Asp Ser Ser His Ser Asp Tyr Leu Leu Thr Ile
       70                  75                  80
   tcc gtt aag acg tca gct gga ccg act aac ctg cgg att gag tac caa   522
   Ser Val Lys Thr Ser Ala Gly Pro Thr Asn Leu Arg Ile Glu Tyr Gln
   85                  90                  95                  100
   gat ggg aaa ttc aga ttg gat tct atc ata tgt gtc aag tcc aag ctt   570
   Asp Gly Lys Phe Arg Leu Asp Ser Ile Ile Cys Val Lys Ser Lys Leu
                   105                 110                 115
   aaa cag ttt gac agt gtg gtt cat ctg att gac tac tat gtc cag atg   618
   Lys Gln Phe Asp Ser Val Val His Leu Ile Asp Tyr Tyr Val Gln Met
               120                 125                 130
   tgc aag gat aaa cgg aca ggc cca gaa gcc cca cgg aat ggg act gtt   666
   Cys Lys Asp Lys Arg Thr Gly Pro Glu Ala Pro Arg Asn Gly Thr Val
           135                 140                 145
   cac ctg tac ctg acc aaa cct ctg tat aca tca gca ccc act ctg cag    714
   His Leu Tyr Leu Thr Lys Pro Leu Tyr Thr Ser Ala Pro Thr Leu Gln
       150                 155                 160
   cat ttc tgt cga ctc gcc att aac aaa tgt acc ggt acg atc tgg gga    762
   His Phe Cys Arg Leu Ala Ile Asn Lys Cys Thr Gly Thr Ile Trp Gly
   165                 170                 175                 180
   ctg cct tta cca aca aga cta aaa gat tac ttg gaa gaa tat aaa ttc    810
   Leu Pro Leu Pro Thr Arg Leu Lys Asp Tyr Leu Glu Glu Tyr Lys Phe
                   185                 190                 195
   cag gta taagtatttc tctctctttt tcgttttttt ttaaaaaaaa aaaaacacat     866
   Gln Val
   gcctcatata gactatctcc gaatgcagct atgtgaaaga gaacccagag gccctcctct 926
   ggataactgc gcagaattct ctcttaagga cagttgggct cagtctaact taaaggtgtg 986
   aagatgtagc taggtatttt aaagttcccc ttaggtagtt ttagctgaat gatgctttct 1046
   ttcctatggc tgctcaagat caaatggccc ttttaaatga aacaaaacaa aacaaaacaa 1106
   aaaaaaaaaa aaaaa                                                  1121
<210> 2
   <211> 198
   <212> PRT
   <213> mouse
   SEQ ID No.2
<400> 2
   Met Thr Leu Arg Cys Leu Glu Pro Ser Gly Asn Gly Ala Asp Arg Thr
    1               5                  10                  15
   Arg Ser Gln Trp Gly Thr Ala Gly Leu Pro Glu Glu Gln Ser Pro Glu
   20 25 30
   Ala Ala Arg Leu Ala Lys Ala Leu Arg Glu Leu Ser Gln Thr Gly Trp
   35 40 45
   Tyr Trp Gly Ser Met Thr Val Asn Glu Ala Lys Glu Lys Leu Lys Glu
   50 55 60
   Ala Pro Glu Gly Thr Phe Leu Ile Arg Asp Ser Ser His Ser Asp Tyr
   65 70 75 80
   Leu Leu Thr Ile Ser Val Lys Thr Ser Ala Gly Pro Thr Asn Leu Arg
   85 90 95
   Ile Glu Tyr Gln Asp Gly Lys Phe Arg Leu Asp Ser Ile Ile Cys Val
   100 105 110
   Lys Ser Lys Leu Lys Gln Phe Asp Ser Val Val His Leu Ile Asp Tyr
   115 120 125
   Tyr Val Gln Met Cys Lys Asp Lys Arg Thr Gly Pro Glu Ala Pro Arg
   130 135 140
   Asn Gly Thr Val His Leu Tyr Leu Thr Lys Pro Leu Tyr Thr Ser Ala
   145 150 155 160
   Pro Thr Leu Gln His Phe Cys Arg Leu Ala Ile Asn Lys Cys Thr Gly
   165 170 175
   Thr Ile Trp Gly Leu Pro Leu Pro Thr Arg Leu Lys Asp Tyr Leu Glu
   180 185 190
   Glu Tyr Lys Phe Gln Val
   195

## Description

### FIELD OF THE INVENTION

The present invention relates generally to the treatment and/or prophylaxis of conditions arising from or otherwise associated with aberrations in hormone signalling. The present invention further provides an animal model useful for screening for agents capable of agonizing or antagonizing hormone signalling. More particularly, the present invention contemplates the treatment and/or prophylaxis of conditions arising from or otherwise associated with aberrations in growth hormone signalling. The present invention further comprises a genetically modified non-human animal. More particularly, the animals are genetically modified such that they have altered growth hormone signalling. The genetically modified non-human animals of the present invention range from laboratory animals useful inter alia for animal models for studying hormone regulation and for the development of therapeutic protocols predicated, in part, on modulating hormone signalling to livestock animals. The latter may be manipulated for improved food production.

### BACKGROUND OF THE INVENTION

The reference to any prior art in this specification is not, and should not be taken as, an acknowledgment or any form of suggestion that that prior art forms part of the common general knowledge in Australia or in any other country.

Bibliographic details of the publications numerically referred to in this specification are collected at the end of the description.

Cells continually monitor their environment in order to modulate physiological and biochemical processes which in turn effects future behaviour. Frequently, a cell's initial interaction with its surroundings occurs via receptors expressed on the plasma membrane. Activation of these receptors, whether through binding endogenous ligands (such as cytokines) or exogenous ligands (such as antigens), triggers a biochemical cascade from the membrane through the cytoplasm to the nucleus.

Of the endogenous ligands, cytokines represent a particularly important and versatile group. Cytokines are proteins which regulate the survival, proliferation, differentiation and function of a variety of cells within the body (1). The haemopoietic cytokines have in common a four-alpha helical bundle structure and the vast majority interact with a structurally related family of cell surface receptors, the type I and type II cytokine receptors (2, 3). In all cases, ligand-induced receptor aggregation appears to be a critical even in initiating intracellar signal transduction cascades. Some cytokines, for example, growth hormone, erythropoietin (Epo) and granulocyte-colony-stimulating factor (G-CSF), trigger receptor homodimerization, while for other cytokines, receptor heterodimerization or heterotrimerization is crucial. In the latter cases, several cytokines share common receptor subunits and on this basis can be grouped into three subfamilies with similar patterns of intracellular activation and similar biological effects (4). Interleukin-3 (IL-3), IL-5 and granulocyte-macrophage colony-stimulating factor (GM-CSF) use the common β-receptor subunit (βc) and each cytokine stimulates the production and functional activity of granulocytes and macrophages. IL-2, IL-4, IL-7, IL-9, and IL-15 each use the common γ-chain (γc), while IL-4 and IL-13 share an alternative γ-chain (γ'c or IL-13 receptor α-chain). Each of these cytokines plays an important role in regulating acquired immunity in the lymphoid system. Finally, IL-6, IL-11, leukemia inhibitory factor (LIF), oncostatin (OSM), ciliary neurotrophic factor (CNTF) and cardiotrophin (CT) share the receptor subunit gp130. Each of these cytokines appears to be highly pleiotropic, having effects both within and outside the haemopoietic system (1).

In all of the above cases, at least one subunit of each receptor complex contains the conserved sequence elements, termed box 1 and box 2, in their cytoplasmic tails (5). Box 1 is a proline-rich motif which is located more proximal to the transmembrane domain than the acidic box 2 element. The box-1 region serves as the binding site for a class of cytoplasmic tyrosine kinases termed JAKs (Janus kinases). Ligand-induced receptor dimerization serves to increase the catalytic activity of the associated JAKs through cross-phosphorylation. Activated JAKs then tryosine phosphorylate several substrates, including the receptors themselves. Specific phosphotyrosine residues on the receptor then serve as docking sites for SH2-containing proteins, the best characterized of which are the signal transducers and activators of transcription (STATs) and the adaptor protein, shc. The STATs are then phosphorylated on tyrosines, probably by JAKs, dissociate from the receptor and form either homodimers or heterodimers through the interaction of the SH2 domain of one STAT with the phosphotyrosine residue of the other. STAT dimers then translocate to the nucleus where they bind to specific cytokine-responsive promoters and activate transcription (6, 7, 8). In a separate pathway, tyrosine phosphorylated shc interacts with another SH2 domain-containing protein, Grb-2 leading ultimately to activation of members of the MAP kinase family and in turn transcription factors such as fos and jun (9, 10). These pathways are not unique to members of the cytokine receptor family since cytokines that bind receptor tyrosine kinases also being able to activate STATs and members of the MAP kinase family (9, 10, 11, 12).

Four members of the JAK family of cytoplasmic tyrosine kinases have been described, JAK1, JAK2, JAK3 and TYK2, each of which binds to a specific subset of cytokine receptor subunits. Six STATs have been described (STAT1 through STAT6), and these too are activated by distinct cytokine/receptor complexes. For example, STAT1 appears to be functionally specific to the interferon system, STAT4 appears to be specific to IL-12, while STAT6 appears to be specific for IL-4 and IL-13. Thus, despite common activation mechanism some degrees of cytokine specificity may be achieved through the use of specific JAKs and STATs.

In addition to those described above, there are clearly other mechanisms of activation of these pathways. For example, the JAK/STAT pathway appears to be able to activate MAP kinases independent of the shc-induced pathway (13) and the STATs themselves can be activated without binding to the receptor, possibly by direct interaction with JAKs (14). Conversely, full activation of STATs may require the action of MAP kinase in addition to that of JAKs (13, 15).

While activation of these signalling pathways is becoming better understood, little is known of the regulation of these pathways, including employment of negative or positive feedback loops. This is important since once a cell has begun to respond to a stimulus, it is critical that the intensity and duration of the response is regulated and that signal transduction is switched off. It is likewise desirable to increase the intensity of a response systemically or even locally as the situation requires.

In International Patent Application No. PCT/AU97/00729 [WO 98/20023], a new family of negative regulators of signal transduction were identified. The new family is the "suppressor of cytokine signalling" or "SOCS" family. It has now been surprisingly determined that modulating the levels of expression of SOCS genes in animal models has an effect on hormone and in particular growth hormone signalling.

Growth hormone modification has been shown to be beneficial in medicine and in the maintenance of domestic livestock (Thomer et al., 1997. Recent Progress in Hormone Research, 52 : 215 and Ward et al., 1998. Reprod. Fertil. Dev., 10 : 659.) Furthermore, molecular interactions between growth hormone and SOCS-2 (Favre et al., 1999. FEBS Letters, 453 : 63) and IGF-I receptor and SOCS-2 (Dey et al., 1998, J. Biol. Chem., 273 : 24098) have been disclosed.

### SUMMARY OF THE INVENTION

Nucleotide and amino acid sequences are referred to by a sequence identifier, i.e. SEQ ID No. 1, SEQ ID No. 2, etc. A sequence listing is provided after the claims.

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

We describe a method for modulating hormone signalling in an animal, said method comprising up-regulating or down-regulating expression of a genetic sequence encoding a SOCS protein or its derivative or homologue or increasing or decreasing the activity of a SOCS protein or its derivative or homologue in said animal.

A first aspect of the present invention, provides a non-medical method of modulating hormone signalling in a livestock or laboratory test animal, said method comprising up-regulating or down-regulating expression of a genetic sequence encoding a SOCS-2 protein or increasing or decreasing the activity of a SOCS-2 protein in said animal and wherein said SOCS-2 protein comprises a protein : molecule interacting region such as but not limited to an SH2 domain,
WD-40 repeats and/or ankyrin repeats, N terminal of a SOCS box, wherein said SOCS box comprises the amino acid sequence:
X₁ X₂ X₃ X₄ X₅ X₆ X₇ X₈ X₉ X₁₀ X₁₁ X₁₂ X₁₃ X₁₄ X₁₅ X₁₆ [Xᵢ]ₙ X₁₇ X₁₈ X₁₉ X₂₀ X₂₁ X₂₂ X₂₃ [Xⱼ]ₙ X₂₄ X₂₅ X₂₆ X₂₇ X₂₈
wherein:
X₁ is L, I, V, M, A or P;
X₂ is any amino acid residue;
X₃ is P, T or S;
X₄ is L, I, V, M, A or P;
X₅ is any amino acid;
X₆ is any amino acid;
X₇ is L, I, V, M, A, F, Y or W;
X₈ is C, T or S;
X₉ is R, K or H;
X₁₀ is any amino acid;
X₁₁ is any amino acid;
X₁₂ is L, I, V, M, A or P;
X₁₃ is any amino acid;
X₁₄ is any amino acid;
X₁₅ is any amino acid;
X₁₆ is L, I, V, M, A, P, G, C, T or S;
[Xᵢ]ₙ is a sequence of n amino acids wherein n is from 1 to 50 amino acids and wherein the sequence Xᵢ may comprise the same or different amino acids selected from any amino acid residue;
X₁₇ is L, I, V, M, A or P;
X₁₈ is any amino acid;
X₁₉ is any amino acid;
X₂₀ is L, I, V, M, A or P;
X₂₁ is P;
X₂₂ is L, I, V, M, A, P or G;
X₂₃ is P or N;
[Xⱼ]ₙ is a sequence of n amino acids wherein n is from 0 to 50 amino acids and wherein the Xⱼ may comprise the same or different amino acids selected from any amino acid residue;
X₂₄ is L, I, V, M, A or P;
X₂₅ is any amino acid;
X₂₆ is any amino acid;
X₂₇ is Y or F;
X₂₈ is L, I, V, M, A or P,
and wherein said SOCS-2 protein comprises the amino acid sequence set forth in SEQ ID NO: 2 or an amino acid sequence having at least 60% identity thereto.

Still another aspect of the present invention contemplates a non-medical method for controlling hormone signalling such as growth hormone signalling in a livestock or laboratory test animal, said method comprising up-regulating or down-regulating expression of a genetic sequence encoding a SOCS-2 protein comprising a SOCS box and a protein : molecule interacting region N-terminal of said SOCS box wherein said SOCS box comprises the amino acid sequence:
X₁ X₂ X₃ X₄ X_{5 X6} X₇ X₈ X₉ X₁₀ X₁₁ X₁₂ X₁₃ X₁₄ X₁₅ X₁₆ [Xᵢ]ₙ X₁₇ X₁₈ X₁₉ X₂₀ X₂₁ X₂₂ X₂₃ [Xⱼ]ₙ X₂₄ X₂₅ X₂₆ X₂₇ X₂₈
wherein:
X₁ is L, I, V, M, A or P;
X₂ is any amino acid residue;
X₃ is P, T or S;
X₄ is L, I, V, M, A or P;
X₅ is any amino acid;
X₆ is any amino acid;
X₇ is L, I, V, M, A, F, Y or W;
X₈ is C, T or S;
X₉ is R, K or H;
X₁₀ is any amino acid;
X₁₁ is any amino acid;
X₁₂ is L, I, V, M, A or P;
X₁₃ is any amino acid;
X₁₄ is any amino acid;
X₁₅ is any amino acid;
X₁₆ is L, I, V, M, A, P, G, C, T or S;
[Xᵢ]ₙ is a sequence of n amino acids wherein n is from 1 to 50 amino acids and wherein the sequence Xᵢ may comprise the same or different amino acids selected from any amino acid residue;
X₁₇ is L, I, V, M, A or P;
X₁₈ is any amino acid;
X₁₉ is any amino acid;
X₂₀ is L, I, V, M, A or P;
X₂₁ is P;
X₂₂ is L, I, V, M, A, P or G;
X₂₃ is P or N;
[Xⱼ]ₙ is a sequence ofn amino acids wherein n is from 1 to 50 amino acids and wherein the Xⱼ may comprise the same or different amino acids selected from any amino acid residue;
X₂₄ is L, I, V, M, A or P;
X₂₅ is any amino acid;
X₂₆ is any amino acid;
X₂₇ is Y or F;
X₂₈ is L, I, V, M, A or P.
and wherein said SOCS-2 protein comprises the amino acid sequence set forth in SEQ ID NO: 2 or an amino acid sequence having at least 60% identity thereto.

Yet another aspect of the present invention contemplates a non-medical method for controlling growth hormone signalling in livestock or laboratory test animal, said method comprising administering to said animal a control-effective amount of a SOCS-2 protein as defined above or functional part or homologue or analogue thereof or antisense RNA or I DNA, ribozymes or sense molecules that manipulate expression of SOCS-2 genes or translation of SOCS-2 mRNA wherein said SOCS-2 gene and said SOCS-2 mRNA encode a SOCS-2 protein or derivative or homologue as defined above, for a time and under conditions sufficient to modulate growth hormone signalling.

Non-medical methods in other species, and medical and non-medical methods involving SOCS proteins other than SOCS-2 are also described.

Genetically modified animals such as livestock animals can be made.

Also described is a genetically modified animal exhibiting altered hormone and in particular growth hormone signalling.

The present invention provides a genetically modified non-human animal as defined in claims 1 to 5. For example, the animal, before genetic modification, may comprise a genetic sequence which comprises a sequence of nucleotides substantially corresponding to the nucleotide sequence set forth in SEQ ID No. 1 or its complementary form or is able to hybridize under low stringency conditions at 42°C to SEQ ID No. 1 or its complementary form and wherein said genetic modification either results in a deletion of one or more nucleotides from said genetic sequence from said animal or substantially prevents, reduces or down-regulates expression of said genetic sequence.

Alternatively said a genetically modified non-human animal may comprise a substantial deletion in a genetic sequence comprising a nucleotide sequence substantially corresponding to SEQ ID No. 1 or capable of hybridizing to SEQ ID No. 1 under low stringency conditions at 42°C and wherein said animal exhibits altered hormone regulation such as growth hormone regulation.

### BRIEF DESCRIPTION OF THE FIGURES

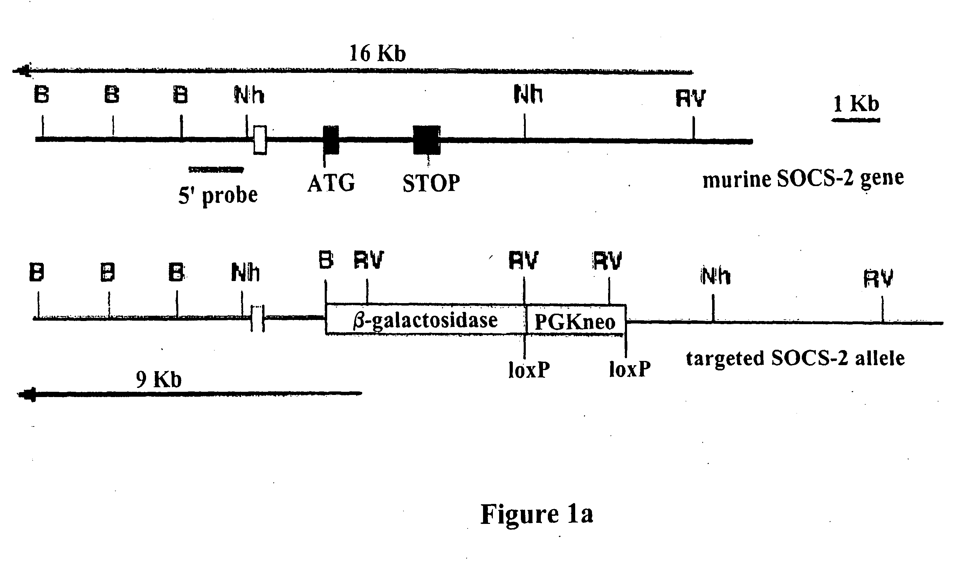

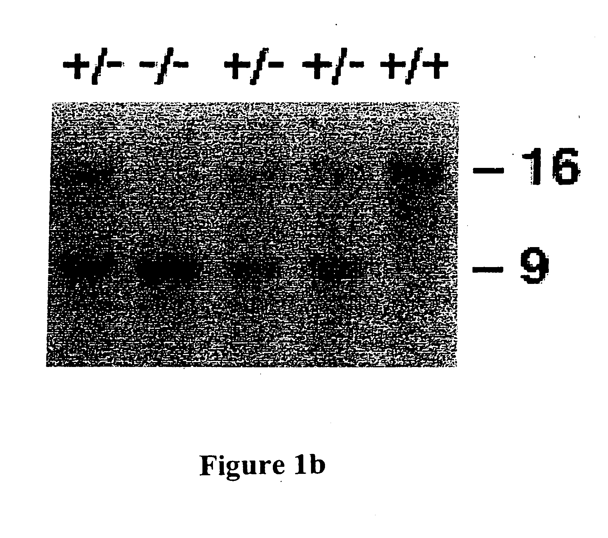

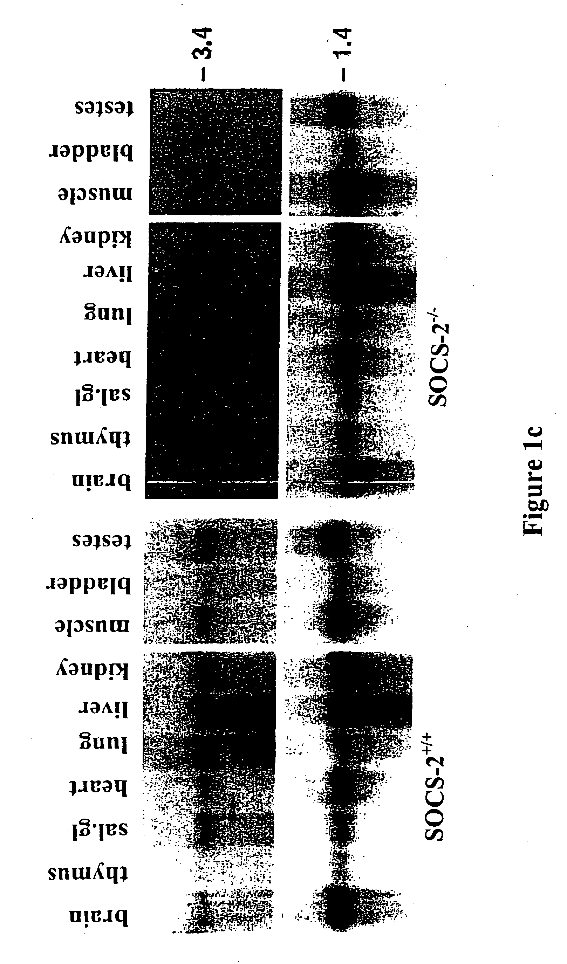

**Figure 1** is a diagrammatic representation showing disruption of the SOCS-2 gene by homologous recombination. (a) The functional murine SOCS-2 gene (B, *Bam*HI; Nh, *Nhe*I; RV. *Eco*RV) with the exons containing the coding region as shaded boxes. In the targeted allele, the entire SOCS-2 coding region was replaced by a β-gal-PGKneo cassette in which the β-galactosidase coding region was fused to the SOCS-2 initiation codon. (b) Southern blot of *Eco*RV-digested genomic DNA from the tails of mice derived from a cross beween SOCS-2^{+/-} mice. The blot was hybridized with the 5' genomic SOCS-2 probe, which distinguishes between endogenous (16 kb) and mutant SOCS-2 (9 kb) alleles. (c) Northern blot showing lack of SOCS-2 expression in organs of SOCS-2^{-/-} mice. Top, the blot was hybridized with a coding region probe, which detects the 3.4-kb SOCS-2 transcript: bottom, the integrity of the RNA was confirmed by hybridization with GAPDH (1.4-kb transcript). Sal gl, salivary gland.

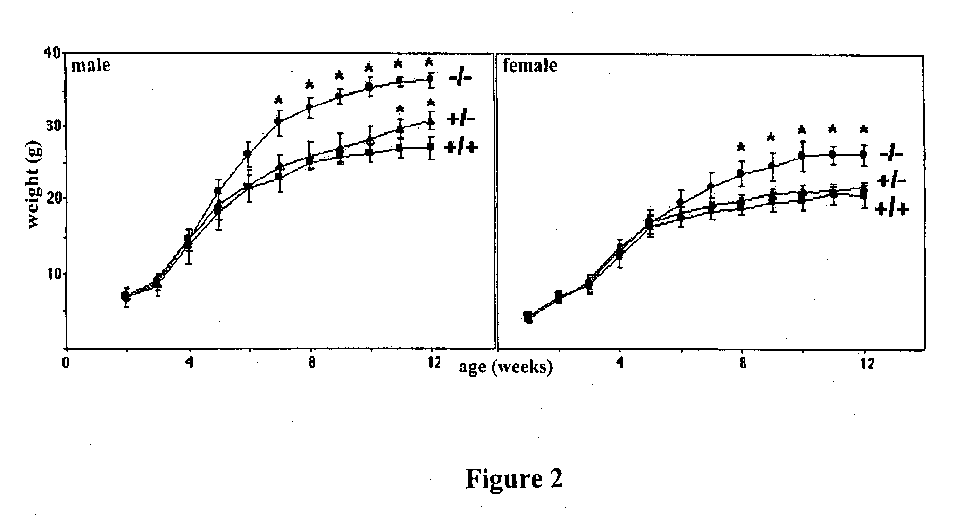

**Figure 2** is a graphical representation showing excessive growth of SOCS-2^{-/-} mice. Growth curves for male and female SOCS-2^{+/+} (squares), SOCS-2^{+/-} (triangles) and SOCS-2^{-/-} (circles) mice. Body weights from cohorts of mice weighed at weekly intervals are shown: each point represents mean + s.d. for 5-20 mice.

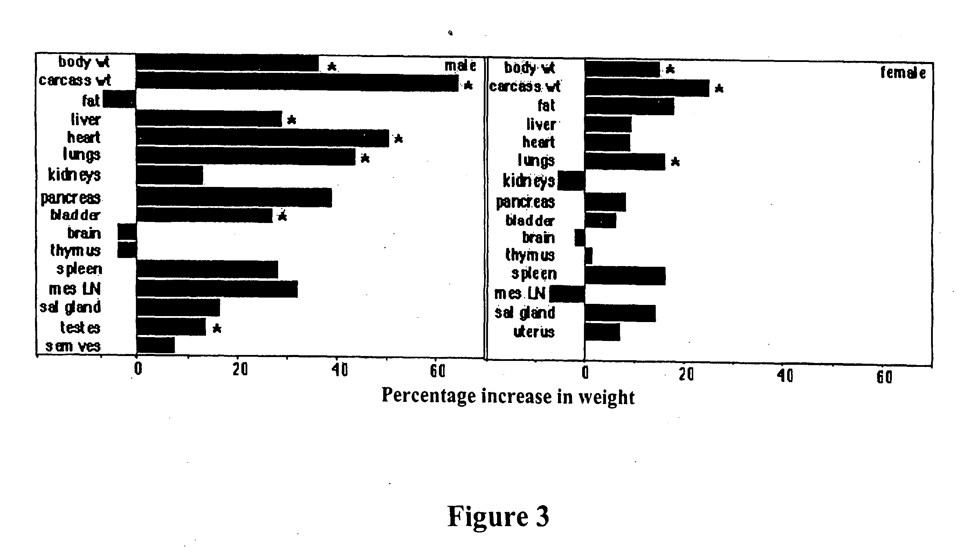

**Figure 3** is a graphical representation showing percentage increase in body, carcass and organ weights in SOCS-2^{-/-} mice over that determined in age-matched wild-type mice (*N* = 7-8 three-month-old mice per measurement). *, measurements from SOCS-2^{-/-} mice that were significantly different from wild-type controls (P <0.05, Student's *t*-test). wt, weight; mes LN, mesenteric lymph node; sal gland, salivary gland; sem ves, seminal vesicles.

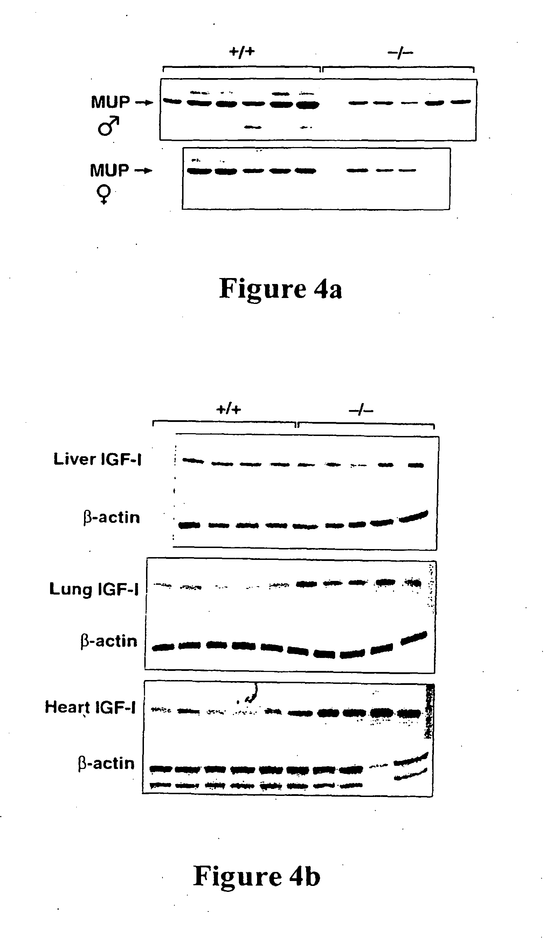

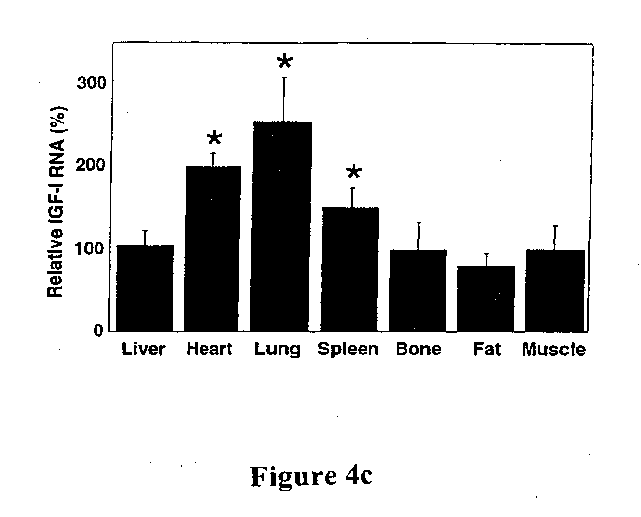

**Figure 4** is a representation showing deregulated growth hormone signalling in SOCS-2^{-/-} mice. (a) Decreased MUP in urine samples from male and female SOCS-2^{-/-} mice. Each lane represents a sample from an individual SOCS-2^{-/-} or age and sex-matched wild-type mouse. (b) RNase protection assays of IGF-I expression in tissues of SOCS-2^{-/-} mice. Each lane represents a sample from an individual SOCS-2^{-/-} or age and sex-matched wild-type mouse. (c) Expression of IGF-I RNA is tissues of male SOCS-2^{-/-} mice, expressed as a percentage of expression in age and sex-matched wild type mice. Data represents the mean and standard deviation for comparison of at least 4 pairs of mice, * p< 0.05.

### DETAILED DESCRIPTION OF THE PREFERRED EMBODIMENTS

One aspect of the present invention contemplates a method for modulating hormone signalling in an animal, said method comprising up-regulating or down-regulating expression of a genetic sequence encoding a SOCS protein or its derivative or homologue or increasing or decreasing the activity of a SOCS protein or its derivative or homologue in said animal.

Reference herein to "SOCS" encompasses any or all members of the SOCS family. Specific SOCS molecules may be defined numerically such as, for example, SOCS-1, SOCS-2 and SOCS-3. The species from which the SOCS has been obtained may be indicated by a preface of single letter abbreviation where "h" is human, "m" is murine and "r" is rat. Accordingly, "mSOCS-2", for example, is a specific SOCS from a murine animal. Reference herein to "SOCS" is not to imply that the protein solely suppresses cytokine-mediated signal transduction, as the molecule may modulate other effector-mediated signal transductions such as by hormones or other endogenous or exogenous molecules, antigen, microbes and microbial products, viruses or components thereof, ions, hormones and parasites. The term "modulates" encompass up-regulation, down-regulation as well as maintenance of particular levels.

Reference herein to a "hormone" includes protein hormones and cytokines as well as non-proteinaceous hormones. One particularly useful hormone is growth hormone. Another useful hormones are insulin-like growth factor I (IGF-I) and prolactin.

An "animal" is preferably a mammal such as but not limited to a human, primate, livestock animal (e.g. sheep, cow, pig, horse, donkey), laboratory test animal (e.g. rabbit, mouse, rat, guinea pig), companion animal (e.g. cat, dog) or captive wild animal. The animal may be in the form of an animal model. Useful animals for this purpose are laboratory test animals. Genetically modifying livestock animals is useful in assisting in food production. The preferred animal is a human, primate animal or laboratory test animal. The most preferred animal is a human.

Reference herein to "SOCS" includes a protein comprising a SOCS box in its C-terminal region comprising the amino acid sequence:
X₁ X₂ X₃ X₄ X₅ X₆ X₇ X₈ X₉ X₁₀ X₁₁ X₁₂ X₁₃ X₁₄ X₁₅ X₁₆ [Xᵢ]ₙ X₁₇ X₁₈ X₁₉ X₂₀ X₂₁ X₂₂ X₂₃ [Xⱼ]ₙ X₂₄ X₂₅ X₂₆ X₂₇ X₂₈
wherein:
X₁ is L, I, V, M, A or P;
X₂ is any amino acid residue;
X₃ is P, T or S;
X₄ is L, I, V, M, A or P;
X₅ is any amino acid;
X₆ is any amino acid;
X₇ is L, I, V, M, A, F, Y or W;
X₈ is C, T or S;
X₉ is R, K or H;
X₁₀ is any amino acid;
X₁₁ is any amino acid;
X₁₂ is L, I, V, M, A or P;
X₁₃ is any amino acid;
X₁₄ is any amino acid;
X₁₅ is any amino acid;
X₁₆ is L, I, V, M, A, P, G, C, T or S;
[Xᵢ]ₙ is a sequence of n amino acids wherein n is from 1 to 50 amino acids and wherein the sequence Xᵢ may comprise the same or different amino acids selected from any amino acid residue;
X₁₇ is L, I, V, M, A or P;
X₁₈ is any amino acid;
X₁₉ is any amino acid;
X₂₀ is L, I, V, M, A or P;
X₂₁ is P;
X₂₂ is L, I, V, M, A, P or G;
X₂₃ is P or N;
[Xⱼ]ₙ is a sequence of n amino acids wherein n is from 0 to 50 amino acids and wherein the Xⱼ may comprise the same or different amino acids selected from any amino acid residue;
X₂₄ is L, L V, K A or P;
X₂₅ is any amino acid;
X₂₆ is any amino acid;
X₂₇ is Y or F;
X₂₈ is L, I, V, M, A or P.

The SOCS protein also comprises a protein:molecule interacting region such as but not limited to one or more of an SH2 domain, WD-40 repeats and/or ankyrin repeats, N-terminal of the SOCS box.

Modulating hormone signalling includes modulating growth control mechanisms. In addition, modulating growth hormone signalling has applications in increasing strength in elderly people, obesity control treatment of catabolic diseases, treatment of chronic inflammatory disease, treatment and/or prophylaxis of osteoporosis, treatment of cardiomyopathy and the treatment of complicated fracture.

The use of SOCS-2 protein as defined above, or a derivative, homologue thereof in the manufacture of a medicament for controlling hormone signalling in the animal as defined in claim 1 is also provided, as is the use of antisense RNA or DNA, ribozymes or sense molecules that manipulate expression of SOCS-2 genes or translation of SOCS-2 mRNA in the manufacture of a medicament for controlling growth hormone signalling in an animal wherein said SOSC-2 gene and said SOCS-2 mRNA encode a SOCS-2 protein or derivative or homologue thereof as defined above.

The present application also describes a method of modulating hormone signalling in an animal and in particular a human, said method comprising up-regulating or down-regulating expression of a genetic sequence encoding a SOCS protein or increasing or decreasing the activity of a SOCS protein in said animal and wherein said SOCS protein comprises a protein : molecule interacting region such as but not limited to an SH2 domain, WD-40 repeats and/or ankyrin repeats, N terminal of a SOCS box, wherein said SOCS box comprises the amino acid sequence:
X₁ X₂ X₃ X₄ X₅ X₆ X₇ X₈ X₉ X₁₀ X₁₁ X₁₂ X₁₃ X₁₄ X₁₅ X₁₆ [Xᵢ]ₙ X₁₇ X₁₈ X₁₉ X₂₀ X₂₁ X₂₂ X₂₃ [Xⱼ]ₙ X₂₄ X₂₅ X₂₆ X₂₇ X₂₈
wherein:
X₁ is L, I, V, M, A or P;
X₂ is any amino acid residue;
X₃ is P, T or S;
X₄ is L, I, V, M, A or P;
X₅ is any amino acid;
X₆ is any amino acid;
X₇ is L, I, V, M, A, F, Y or W;
X₈ is C, T or S;
X₉ is R, K or H;
X₁₀ is any amino acid;
X₁₁ is any amino acid;
X₁₂ is L, I, V, M, A or P;
X₁₃ is any amino acid;
X₁₄ is any amino acid;
X₁₅ is any amino acid;
X₁₆ is L, I, V, M, A, P, G, C, T or S;
[Xᵢ]ₙ is a sequence of n amino acids wherein n is from 1 to 50 amino acids and wherein the sequence Xᵢ may comprise the same or different amino acids selected from any amino acid residue;
X₁₇ is L, I, V, M, A or P;
X₁₈ is any amino acid;
X₁₉ is any amino acid;
X₂₀ is L, I, V, M, A or P;
X₂₁ is P;
X₂₂ is L, I, V, M, A, P or G;
X₂₃ is P or N;
[Xⱼ]ₙ is a sequence of n amino acids wherein n is from 0 to 50 amino acids and wherein the Xⱼ may comprise the same or different amino acids selected from any amino acid residue;
X₂₄ is L, I, V, M, A or P;
X₂₅ is any amino acid;
X₂₆ is any amino acid;
X₂₇ is Y or F;
X₂₈ is L, I, V, M, A or P.

Various SOCS molecules are disclosed in International Patent Application No. PCT/AU99/00729 [WO 98/20023]. The present invention is directed to manipulating levels of SOCS-2, which murine form comprises the nucleotide and corresponding amino acid sequence as set forth in SEQ ID No. 1 and SEQ ID No. 2, respectively. The present invention is hereinafter described with reference to murine
SOCS-2 (mSOCS-2), however, this is done with the understanding that the present invention encompasses the manipulation of levels of any SOCS-2 molecule, such as but not limited to human SOCS-2 (hSOCS-2). It is also possible to manipulate the levels of other SOCS molecules. The nucleotide sequence of hSOCS-2 and its corresponding amino acid sequence can be found at Gen Bank Accession Number
AF037989 (see also reference 19). Reference herein to a "SOCS" molecule such as SOCS-2 includes any mutants thereof such as functional mutants. An example of a mutant is a single or multiple amino acid substitution, addition and/or deletion or truncation to the SOCS molecule or its corresponding DNA or RNA. Another useful SOCS molecule is SOCS-3.

The present invention is predicated in part on the determination that knock out mice for a SOCS gene exhibit altered hormone signalling. In particular, mice which substantially do not produce SOCS-2 exhibit disrupted growth hormone signalling and, as a result, have a growth advantage over their littermates.

This provides the basis for developing therapeutic protocols for subjects which either require more or require less growth hormone signalling. It also has important implications in the veterinary industry for manipulating animals to provide same with a growth advantage. This may be important for food production. Alternatively, where a reduction in the size of an animal is desired, facilitating expression of a SOCS gene or facilitating SOCS protein activity will result in greater growth hormone signalling.

Although the present invention is specifically exemplified in relation to growth hormone, the instant invention extends to any hormone signalling such as facilitated by protein hormones (i.e. cytokines) and non-protenaceous hormones.

The present invention provides a non-medical method for controlling growth hormone signalling in a livestock or laboratory test animal, said method comprising upregulating or downregulating expression of a genetic sequence encoding a SOCS-2 protein comprising a SOCS box and a protein : molecule interacting region N-terminal of said SOCS box wherein said SOCS box comprises the amino acid sequence:
X₁ X₂ X₃ X₄ X₅ X₆ X₇ X₈ X₉ X₁₀ X₁₁ X₁₂ X₁₃ X₁₄ X₁₅ X₁₆ [Xᵢ]ₙ X₁₇ X₁₈ X₁₉ X₂₀ X₂₁ X₂₂ X₂₃ [Xⱼ]ₙ X₂₄ X₂₅ X₂₆ X₂₇ X₂₈
wherein:
X₁ is L, I, V, M, A or P;
X₂ is any amino acid residue;
X₃ is P, T or S;
X₄ is L, I, V, M, A or P;
X₅ is any amino acid;
X₆ is any amino acid;
X₇ is L, I, V, M, A, F, Y or W;
X₈ is C, T or S;
X₉ is R, K or H;
X₁₀ is any amino acid;
X₁₁ is any amino acid;
X₁₂ is L, I, V, M, A or P;
X₁₃ is any amino acid;
X₁₄ is any amino acid;
X₁₅ is any amino acid;
X₁₆ is L, I, V, M, A, P, G, C, T or S;
[Xᵢ]ₙ is a sequence of n amino acids wherein n is from 1 to 50 amino acids and wherein the sequence Xᵢ may comprise the same or different amino acids selected from any amino acid residue;
X₁₇ is L, I, V, M, A or P;
X₁₈ is any amino acid;
X₁₉ is any amino acid;
X₂₀ is L, I, V, M, A or P;
X₂₁ is P;
X₂₂ is L, I, V, M, A, P or G;
X₂₃ is P or N;
[Xⱼ]ₙ is a sequence of n amino acids wherein n is from 0 to 50 amino acids and wherein the Xⱼ may comprise the same or different amino acids selected from any amino acid residue;
X₂₄ is L, I, V, M, A or P;
X₂₅ is any amino acid;
X₂₆ is any amino acid;
X₂₇ is Y or F;
X₂₈ is L, I, V, M, A or P,
and wherein the SOCS protein-encoding genetic sequence comprises a nucleotide sequence set forth in SEQ ID No. 1 or a nucleotide sequence having at least 60% similarity thereto. Preferably the nucleotide sequence is capable of hybridizing to SEQ ID No. 1 or its complementary form under low stringency conditions at 42°C. Even more preferably, the SOCS protein in a human homologue of the nucleotide sequence set forth in SEQ ID No. 1.

Non-medical methods for controlling growth hormone signalling for other animals are also described, as are medical methods for controlling growth hormone signalling, and non-medical and medical methods for controlling hormone signalling in any animal. In these methods, the expression of SOCS-2. or other SOCS molecules as defined herein may be modulated.

The term "similarity" as used herein includes exact identity between compared sequences at the nucleotide or amino acid level. Where there is non-identity at the nucleotide level, "similarity" includes differences between sequences which result in different amino acids that are nevertheless related to each other at the structural, functional, biochemical and/or conformational levels. Where there is non-identity at the amino acid level, "similarity" includes amino acids that are nevertheless related to each other at the structural, functional, biochemical and/or conformational levels. In a particularly preferred embodiment, nucleotide and sequence comparisons are made at the level of identity rather than similarity.

Terms used to describe sequence relationships between two or more polynucleotides or polypeptides include "reference sequence", "comparison window", "sequence similarity", "sequence identity", "percentage of sequence similarity", "percentage of sequence identity", "substantially similar" and "substantial identity". A "reference sequence" is at least 12 but frequently 15 to 18 and often at least 25 or above, such as 30 monomer units, inclusive of nucleotides and amino acid residues, in length. Because two polynucleotides may each comprise (1) a sequence (i.e. only a portion of the complete polynucleotide sequence) that is similar between the two polynucleotides, and (2) a sequence that is divergent between the two polynucleotides, sequence comparisons between two (or more) polynucleotides are typically performed by comparing sequences of the two polynucleotides over a "comparison window" to identify and compare local regions of sequence similarity. A "comparison window" refers to a conceptual segment of typically 12 contiguous residues that is compared to a reference sequence. The comparison window may comprise additions or deletions (i.e. gaps) of about 20% or less as compared to the reference sequence (which does not comprise additions or deletions) for optimal alignment of the two sequences. Optimal alignment of sequences for aligning a comparison window may be conducted by computerised implementations of algorithms (GAP, BESTFIT, FASTA, and TFASTA in the Wisconsin Genetics Software Package Release 7.0, Genetics Computer Group, 575 Science Drive Madison, WI, USA) or by inspection and the best alignment (i.e. resulting in the highest percentage homology over the comparison window) generated by any of the various methods selected. Reference also may be made to the BLAST family of programs as, for example, disclosed by Altschul *et al.* (16). A detailed discussion of sequence analysis can be found in Unit 19.3 of Ausubel *et al.* (17).

The terms "sequence similarity" and "sequence identity" as used herein refers to the extent that sequences are identical or functionally or structurally similar on a nucleotide-by-nucleotide basis or an amino acid-by-amino acid basis over a window of comparison. Thus, a "percentage of sequence identity", for example, is calculated by comparing two optimally aligned sequences over the window of comparison, determining the number of positions at which the identical nucleic acid base (e.g. A, T, C, G, I) or the identical amino acid residue (e.g. Ala, Pro, Ser, Thr, Gly, Val, Leu, Ile, Phe, Tyr, Trp, Lys, Arg, His, Asp, Glu, Asn, Gin, Cys and Met) occurs in both sequences to yield the number of matched positions, dividing the number of matched positions by the total number of positions in the window of comparison (i.e., the window size), and multiplying the result by 100 to yield the percentage of sequence identity. For the purposes of the present invention, "sequence identity" will be understood to mean the "match percentage" calculated by the DNASIS computer program (Version 2.5 for windows; available from Hitachi Software engineering Co., Ltd., South San Francisco, California, USA) using standard defaults as used in the reference manual accompanying the software. Similar comments apply in relation to sequence similarity.

Reference herein to a low stringency includes and encompasses from at least about 0 to at least about 15% v/v formamide and from at least about 1 M to at least about 2 M salt for hybridization, and at least about 1 M to at least about 2 M salt for washing conditions. Generally, low stringency is at from about 25-30°C to about 42°C. The temperature may be altered and higher temperatures used to replace formamide and/or to give alternative stringency conditions. Alternative stringency conditions may be applied where necessary, such as medium stringency, which includes and encompasses from at least about 16% v/v to at least about 30% v/v formamide and from at least about 0.5 M to at least about 0.9 M salt for hybridization, and at least about 0.5 M to at least about 0.9 M salt for washing conditions, or high stringency, which includes and encompasses from at least about 31 % v/v to at least about 50% v/v formamide and from at least about 0.01 M to at least about 0.15 M salt for hybridization, and at least about 0.01 M to at least about 0.15 M salt for washing conditions. In general, washing is carried out Tₘ = 69.3 + 0.41 (G+C)% (18).

However, the Tₘ of a duplex DNA decreases by 1°C with every increase of 1% in the number of mismatch base pairs (19). Formamide is optional in these hybridization conditions. Accordingly, particularly preferred levels of stringency are defined as follows: low stringency is 6 x SSC buffer, 0.1 % w/v SDS at 25-42°C; a moderate stringency is 2 x SSC buffer, 0.1% w/v SDS at a temperature in the range 20°C to 65°C; high stringency is 0.1 x SSC buffer, 0.1% w/v SDS at a temperature of at least 65°C.

In an alternative embodiment, the modulation of growth hormone signalling is accomplished at the protein level using a composition comprising SOCS-2 or its derivative, homologue or analogue. The use of a composition comprising other SOCS proteins, as well as the use of SOCS antagonists or agonists is also described.

Accordingly, another aspect of the present invention contemplates a non-medical method for controlling growth hormone signalling in livestock or laboratory test animal, said method comprising administering to said animal a control-effective amount of a SOCS-2 protein or functional part or homologue or analogue thereof or antisense RNA or DNA, nbozymes or sense molecules to manipulate expression of SOCS-2 genes or translation of SOCS-2 mRNA wherein said SOCS-2 gene and said SOCS m-RNA encode a SOCS-2 protein or derivative or homologue thereof as described above, for a time and under conditions sufficient to modulate growth hormone signalling. Non-medical methods for controlling growth hormone signalling in other animals and non-medical and medical methods for controlling growth hormone signalling using other SOCS proteins or antagonists or agonists thereof are also described.

Where a SOCS protein is administered, it may be an isolated form of the naturally occurring SOCS protein or it may be a derivative, homologue or analogue thereof.

A further aspect contemplates genetically modified animals such as livestock animals. Such animals, having altered hormone signalling, are useful *inter alia* for food production.

A "derivative" includes a part, portion or fragment thereof such as a molecule comprising a single or multiple amino acid substitution, deletion and/or addition. A "homologue" includes a functionally similar molecule from either the same species or another species.

Analogues contemplated herein include, but are not limited to, modification to side chains, incorporating of unnatural amino acids and/or their derivatives during peptide, polypeptide or protein synthesis and the use of cross linkers and other methods which impose conformational constraints on the proteinaceous molecule or their analogues.

Examples of side chain modifications contemplated by the present invention include modifications of amino groups such as by reductive alkylation by reaction with an aldehyde followed by reduction with NaBH₄; amidination with methylacetimidate; acylation with acetic anhydride; carbamoylation of amino groups with cyanate; trinitrobenzylation of amino groups with 2, 4, 6-trinitrobenzene sulphonic acid (TNBS); acylation of amino groups with succinic anhydride and tetrahydrophthalic anhydride; and pyridoxylation of lysine with pyridoxal-5-phosphate followed by reduction with NaBH₄.

The guanidine group of arginine residues may be modified by the formation of heterocyclic condensation products with reagents such as 2,3-butanedione, phenylglyoxal and glyoxal.

The carboxyl group may be modified by carbodiimide activation via O-acylisourea formation followed by subsequent derivitization, for example, to a corresponding amide.

Sulphydryl groups may be modified by methods such as carboxymethylation with iodoacetic acid or iodoacetamide; performic acid oxidation to cysteic acid; formation of a mixed disulphides with other thiol compounds; reaction with maleimide, maleic anhydride or other substituted maleimide; formation of mercurial derivatives using 4-chloromercuribenzoate, 4-chloromercuriphenylsulphonic acid, phenylmercury chloride, 2-chloromercuri-4-nitrophenol and other mercurials; carbamoylation with cyanate at alkaline pH.

Tryptophan residues may be modified by, for example, oxidation with N-bromosuccinimide or alkylation of the indole ring with 2-hydroxy-5-nitrobenzyl bromide or sulphenyl halides. Tyrosine residues on the other hand, may be altered by nitration with tetranitromethane to form a 3-nitrotyrosine derivative.

Modification of the imidazole ring of a histidine residue may be accomplished by alkylation with iodoacetic acid derivatives or N-carbethoxylation with diethylpyrocarbonate.

Examples of incorporating unnatural amino acids and derivatives during peptide synthesis include, but are not limited to, use of norleucine, 4-amino butyric acid, 4-amino-3-hydroxy-5-phenylpentanoic acid, 6-aminohexanoic acid, t-butylglycine, norvaline, phenylglycine, omithine, sarcosine, 4-amino-3-hydroxy-6-methylheptanoic acid, 2-thienyl alanine and/or D-isomers of amino acids. A list of unnatural amino acid, contemplated herein is shown in Table 1.

**TABLE 1**

| Non-conventional amino acid | Code | Non-conventional amino acid | Code |
|---|---|---|---|
| α-aminobutyric acid | Abu | L-N-methylalanine | Nmala |
| α-amino-α-methylbutyrate | Mgabu | L-N-methylarginine | Nmarg |
| aminocyclopropane- | Cpro | L-N-methylasparagine | Nmasn |
| carboxylate | | L-N-methylaspartic acid | Nmasp |
| aminoisobutyric acid | Aib | L-N-methylcysteine | Nmcys |
| aminonorbomyl- | Norb | L-N-methylglutamine | Nmgln |
| carboxylate | | L-N-methylglutamic acid | Nmglu |
| cyclohexylalanine | Chexa | L-Nmethylhistidine | Nmhis |
| cyclopentylalanine | Cpen | L-N-methylisolleucine | Nmile |
| D-alanine | Dal | L-N-methylleucine | Nmleu |
| D-argininc | Darg | L-N-methyllysine | Nmlys |
| D-aspanic acid | Dasp | L-N-methylmethionine | Nmmet |
| D-cysteine | Dcys | L-N-methylnorleucine | Nmnle |
| D-glutamine | Dgln | L-N-methylnorvaline | Nmnva |
| D-glutamic acid | Dglu | L-N-methylornithine | Nmorn |
| D-histidine | Dhis | L-N-methylphenylalanine | Nmphe |
| D-isoleucine | Dile | L-N-methylproline | Nmpro |
| D-leucine | Dleu | L-N-methylserine | Nmser |
| D-lysine | Dlys | L-N-methylthreonine | Nmthr |
| D-methioninc | Dmet | L-N-methyltryptophan | Nmtrp |
| D-ornithine | Dorn | L-N-methyltyrosine | Nmtyr |
| D-phenylalaninc | Dphe | L-N-methylvaline | Nmval |
| D-proline | Dpro | L-N-methylethylglycine | Nmetg |
| D-serine | Dser | L-N-methyl-t-butylglycine | Nmtbug |
| D-threonine | Dthr | L-norleucine | Nle |
| D-tryptophan | Dtrp | L-norvaline | Nva |
| D-tyrosine | Dtyr | α-methyl-aminoisobutyrate | Maib |
| D-valine | Dval | α-methyl-γ-aminobutyrate | Mgabu |
| D-α-methylalanine | Dmala | α-methylcyclohexylalanine | Mchexa |
| D-α-methylarginine | Dmarg | α-methylcylcopentylalanine | Mcpen |
| D-α-methylasparagine | Dmasn | α-methyl-α-napthylalanine | Manap |
| D-α-methylaspartate | Dmasp | α-methylpenicillamine | Mpen |
| D-α-methylcysteine | Dmcys | N-(4-aminobutyl)glycine | Nglu |
| D-α-methylglutamine | Dmgln | N-(2-aminoethyl)glycine | Naeg |
| D-α-methylhistidine | Dmhis | N-(3-aminopropyl)glycine | Nom |
| D-α-methylisoleucine | Dmile | N-amino-α-methylbutyrate | Nmaabu |
| D-α-methylleucine | Dmleu | α-napthylalanine | Anap |
| D-α-methyllysine | Dmlys | N-benzylglycine | Nphe |
| D-α-methylmethionine | Dmmet | N-(2-carbamylethyl)glycine | Ngln |
| D-α-methylornithine | Dmorn | N-(carbamylmethyl)glycine | Nasn |
| D-α-methylphenylalanine | Dmphe | N-(2-carboxyethyl)glycine | Nglu |
| D-α-methylproline | Dmpro | N-(carboxymethyl)glycine | Nasp |
| D-α-methylserine | Dmser | N-cyclobutylglycine | Ncbut |
| D-α-methylthreonine | Dmthr | N-cycloheptylglycine | Nchep |
| D-α-methyltryptophan | Dmtrp | N-cyclohexylglycine | Nchex |
| D-α-methyltyrosine | Dmty | N-cyclodecylglycine | Ncdec |
| D-α-methylvaline | Dmval | N-cylcododecylglycine | Ncdod |
| D-N-methylalanine | Dnmala | N-cyclooctylglycine | Ncoct |
| D-N-methylarginine | Dnmarg | N-cyclopropylglycine | Ncpro |
| D-N-methylasparagine | Dnmasn | N-cycloundecylglycine | Ncund |
| D-N-methylaspartate | Dnmasp | N-(2,2-diphenylethyl)glycine | Nbhm |
| D-N-methylcysteine | Dnmcys | N-(3,3-diphenylpropyl)glycine | Nbhe |
| D-N-methylglutamine | Dnmgln | N-(3-guanidinopropyl)glycine | Narg |
| D-N-methylglutamate | Dnmglu | N-(1-hydroxyethyl)glycine | Nthr |
| D-N-methylhistidine | Dnmhis | N-(hydroxyethyl))glycine | Nser |
| D-N-methylisoleucine | Dnmile | N-(imidazolylethyl))glycine | Nhis |
| D-N-methylleucine | Dnmleu | N-(3-indolylyethyl)glycine | Nhtrp |
| D-N-methyllysine | Dnmlys | N-methyl-γ-aminobutyrate | Nmgabu |
| N-methylcyclohexylalanine | Nmchexa | D-N-methylmethionine | Dnmmet |
| D-N-methylornithine | Dnmorn | N-methylcyclopentylalanine | Nmcpen |
| N-methylglycine | Nala | D-N-methylphenylalanine | Dnmphe |
| N-methylaminoisobutyratc | Nmaib | D-N-methylproline | Dnmpro |
| N-(1-methylpropyl)glycine | Nile | D-N-methylserine | Dnmser |
| N-(2-methylpropyl)glycine | Nleu | D-N-methylthreonine | Dnmthr |
| D-N-methyltryptophan | Dnmtrp | N-(1-methylethyl)glycine | Nval |
| D-N-methyltyrosine | Dnmtyr | N-methyla-napthylalanine | Nmanap |
| D-N-methylvaline | Dnmval | N-methylpenicillamine | Nmpen |
| γ-aminobutyric acid | Gabu | N-(*p*-hydroxyphenyl)glycine | Nhtyr |
| L-*t*-butylglycine | Tbug | N-(thiomethyl)glycine | Ncys |
| L-ethylglycine | Etg | penicillamine | Pen |
| L-homophenylalanine | Hphe | L-α-methylalanine | Mala |
| L-α-methylarginine | Marg | L-α-methylasparagine | Masn |
| L-α-methylaspartate | Masp | L-α-methyl-*t*-butylglycine | Mtbug |
| L-α-methylcysteine | Mcys | L-methylethylglycine | Metg |
| L-a-methylglutamine | Mgln | L-α-methylglutamate | Mglu |
| L-α-methylhistidine | Mhis | L-α-methylhomophenylalanine | Mhphe |
| L-α-methylisoleucine | Mile | N-(2-methylthioethyl)glycine | Nmet |
| L-α-methylleucine | Mleu | L-α-methyllysine | Mlys |
| L-α-methylmethionine | Mmet | L-α-methylnorleucine | Mnle |
| L-α-methylnorvaline | Mnva | L-α-methylornithine | Morn |
| L-α-methylphenylalanine | Mphe | L-α-methylproline | Mpro |
| L-α-methylserine | Mser | L-α-methylthreonine | Mthr |
| L-α-methyltryptophan | Mtrp | L-α-methyltyrosine | Mtyr |
| L-α-methylvaline | Mval | L-N-methylhomophenylalanine | Nmhphe |
| N-(N-(2,2-diplienylethyl) | Nnbhm | N-(N-(3,3-diphenylpropyl) | Nnbhe |
| carbamylmethyl)glycine | | carbamylmethyl)glycine | |
| 1-carboxy-1-(2,2-diphenyl-ethylamino)cyclopropane | Nmbc | | |

Crosslinkers can be used, for example, to stabilize 3D conformations, using homobifunctional crosslinkers such as the bifunctional imido esters having (CH₂)ₙ spacer groups with n = 1 to n = 6, glutaraldehyde, N-hydroxysuccinimide esters and heterobifunctional reagents which usually contain an amino-reactive moiety such as N-hydroxysuccinimide and another group specific-reactive moiety such as maleimido or dithio moiety (SH) or carbodiimide (COOH). In addition, peptides can be conformationally constrained by, for example, incorporation of C_{α} and N_{α}-methylamino acids, introduction of double bonds between C_{α} and C_{β} atoms of amino acids and the formation of cyclic peptides or analogues by introducing covalent bonds such as forming an amide bond between the N and C termini, between two side chains or between a side chain and the N or C terminus.

The present invention further contemplates chemical analogues of SOCS molecules capable of acting as antagonists or agonists of a SOCS or which can act as functional analogues of SOCS. Chemical analogues may not necessarily be derived from SOCS but may share certain conformational similarities. Alternatively, chemical analogues may be specifically designed to mimic certain physiochemical properties of SOCS. Chemical analogues may be chemically synthesized or may be detected following, for example, natural product screening.

Other derivatives contemplated by the present invention include a range of glycosylation variants from a completely unglycosylated molecule to a modified glycosylated molecule. Altered glycosylation patterns may result from expression of recombinant molecules in different host cells.

Compositions such as pharmaceutical compositions comprising one or more of a SOCS protein, a derivative, homologue or analogue thereof and/or an antagonist or agonist of a SOCS protein and one or more pharmaceutically acceptable carriers and/or diluents are also described.

Such compositions are useful for modulating growth hormone signalling in an animal such as a human or livestock animal. Preferably, the composition comprises SOCS-2 or a derivative, homologue or analogue thereof.

In order to facilitate passage of the SOCS protein through various membranes, the molecule may be fused to or co-expressed with TAT, (from HIV) or penetratin. Alternatively, the SOCS protein may be administered following laser treatment.

The preparation of pharmaceutical compositions is well known in the art and is described, for example, in Remington's Pharmaceutical Sciences, Eaton Publishing, Pennsylvania USA or in International Patent Publication No. PCT/AU99/00729 [WO 98/20023].

The present invention also provides for the genetic control of SOCS levels in animals.

For example, compositions comprising antisense RNA or DNA, ribozymes or sense molecules (for co-suppression) may be administered either locally or systemically to manipulate expression of SOCS-2 genes or translation of SOCS-2 mRNA.

Genetically modified animals exhibiting alerted hormone and in particular growth hormone signalling are also described.

Particularly, the present invention is directed to a non-human genetically modified animal exhibiting increased or decreased levels of a SOCS protein or a derivative or homologue thereof wherein the SOCS protein, in a genetically unaltered animal, comprises a protein:molecule interacting portion, N-terminal of a SOCS box, which SOCS box comprises the amino acid sequence:
X₁ X₂ X₃ X₄ X₅ X₆ X₇ X₈ X₉ X₁₀ X₁₁ X₁₂ X₁₃ X₁₄ X₁₅ X₁₆ [Xᵢ]ₙ X₁₇ X₁₈ X₁₉ X₂₀ X₂₁ X₂₂ X₂₃ [Xⱼ]ₙ X₂₄ X₂₅ X₂₆ X₂₇ X₂₈
wherein:
X₁ is L, I, V, M, A or P;
X₂ is any amino acid residue;
X₃ is P, T or S;
X₄ is L, I, V, M, A or P;
X₅ is any amino acid;
X₆ is any amino acid;
X₇ is L, I, V, M, A, F, Y or W;
X₈ is C, T or S;
X₉ is R, K or H;
X₁₀ is any amino acid;
X₁₁ is any amino acid;
X₁₂ is L, I, V, M, A or P;
X₁₃ is any amino acid;
X₁₄ is any amino acid;
X₁₅ is any amino acid;
X₁₆ is L, I, V, M, A, P, G, C, T or S;
[Xᵢ]ₙ is a sequence of n amino acids wherein n is from 1 to 50 amino acids and wherein the sequence Xᵢ may comprise the same or different amino acids selected from any amino acid residue;
X₁₇ is L, I, V, M, A or P;
X₁₈ is any amino acid;
X₁₉ is any amino acid;
X₂₀ is L, I, V, M, A or P;
X₂₁ is P;
X₂₂ is L, I, V, M, A, P or G;
X₂₃ is P or N;
[Xⱼ]ₙ is a sequence of n amino acids wherein n is from 0 to 50 amino acids and wherein the Xⱼ may comprise the same or different amino acids selected from any amino acid residue;
X₂₄ is L, I, V, M, A or P;
X₂₅ is any amino acid;
X₂₆ is any amino acid;
X₂₇ is Y or F;
X₂₈ is L, I, V, M, A or P,
and wherein the SOCS protein is SOCS-2 and comprises the amino acid sequence substantially as set forth in SEQ ID No. 2 or an amino acid sequence having at least 60% similarity thereto.

Animals in which the SOCS protein is a derivative or homologue of SOCS-2 or in which the SOCS protein is another SOCS protein or derivative of homologue thereof are also disclosed.

In a particularly preferred embodiment, the present invention provides a genetically modified non-human animal wherein the animal, before genetic modification, comprises a genetic sequence which comprises a sequence of nucleotides substantially corresponding to the nucleotide sequence set forth in SEQ ID No. 1 or its complementary form or is able to hybridize under low stringency conditions at 42°C to SEQ ID No. 1 or its complementary form and wherein said genetic modification results in a deletion of one or more nucleotides from said genetic sequence from said animal which substantially prevents, reduces or down-regulates expression of said genetic sequence.

The term "expression" includes transcription and/or translation of a genetic sequence.

In a particularly preferred embodiment, the present invention provides a genetically modified non-human animal comprising a substantial deletion in a genetic sequence comprising a nucleotide sequence substantially corresponding to SEQ ID No. 1 or capable of hybridizing to SEQ ID No. 1 under low stringency conditions at 42°C and wherein said animal exhibits altered hormone regulation such as growth hormone regulation.

Genetically modified animals contemplated by the present invention include mice, rats, guinea pigs, rabbits and livestock animals (e.g. pigs, cows, sheep, donkeys, horses). Genetically modified livestock animals are particularly useful in food production.

The genetically modified animals of the present invention are also useful animal models for screening for therapeutic agents capable of modulating growth hormone signalling.

The present invention further extends to agents identified using the animal model of this aspect of the present invention.

The present invention is further described by the following non-limiting Examples.

### EXAMPLE 1

### Generation of ES targeted cells and mutant mice

A genomic SOCS-2 fragment extending approximately 2.0 kb from the protein initiation ATG was generated by PCR. This fragment was fused to the ATG of β-galactosidase *via* the *Bam*HI site in the plasmid vector pßgalpAloxneo (24). The 3' arm, an *Eco*RI fragment extending 3.7 kb downstream from the termination codon was blunted and ligated into the *Xho*I (blunted) site of pßgalpAloxneo that already contained the 5' Arm. This targetting vector was linearized with *Not*I and electroporated into C57BL/6 embryonic stem cells. Transfected cells were selected in G418 and resistant clones picked and expanded. Clones in which the targeting vector had recombined with the endogenous SOCS-2 gene were identified by hybridizing *Eco*RV-digested genomic DNA with a 0.8 kb *Bam*HI-*Nhe*I fragment situated in 5' SOCS-2 genomic sequence just outside the targeting vector (Figure 1). This probe distinguished between the endogenous (16kb) and targeted (9 kb) SOCS-2 alleles. A targeted ES cell clone was injected into Balb/c blastocysts to generate chimaeric mice. Male chimaeras were mated with C57BL/6 females to yield SOCS-2 heterozygotes which were interbred to produce wild-type (SOCS-2^{+/+}), heterozygous (SOCS-2^{+/-}) and mutant (SOCS-2^{-/-}) mice on a pure C57BL/6 genetic background. The genotypes of offspring were determined by Southern Blot analysis of genomic DNA extracted from tail biopsies as described above. The deletion of SOCS-2 coding sequence and subsequent inability to produce SOCS-2 mRNA in mutant mice was confirmed in nucleic acid blots which were performed as previously described (25). Northern blots were probed with a full-length SOCS-2 coding region probe and then with a 1.2kb *Pst*I chicken glyceraldehyde-3-phosphate dehydrogenase (GAPDH) fragment.

### EXAMPLE 2

### Hematological and histological analysis

Peripheral blood white cell and platelet counts were determined manually using haemocytometers. Single cell suspensions from femoral bone marrow, spleen and liver were prepared and differential counts of peripheral blood, bone marrow and spleen were performed from stained smears and cytocentrifuge preparations. Clonal cultures of 2.5 x 10⁴ adult bone marrow cells were performed in 0.3% agar as previously described (26). Cultures were stimulated with recombinant purified GM-CSF, G-CSF, M-CSF, IL-3 (each at 10 ng/ml), SCF (100 ng/ml), IL-6 (100 ng/ml) or Flk-ligand (500 ng/ml) plus LIF (10³ U/ml). Agar cultures were fixed, sequentially stained for acetylcholinesterase, Luxol fast Blue and hematoxylin, and the cellular composition of each colony determined microscopically. Tissue sections were prepared by standard techniques, stained with haematoxylin and eosin and examined by light microscopy.

### EXAMPLE 3

### MUP analysis

To analyze MUP levels, 6-7 week old mice were made to urinate before samples were collected 3 and 5.5 hours later. Samples were pooled and centrifuged (13,000 rpm x 3 min) before 0.5 µl of supernatant was electorphoresed in 12% SDS-polyacrylamide gels and stained with Coomassie blue.

### EXAMPLE 4

### Growth curves and linear measurements

Cohorts of mice were weighed at weekly intervals for 12 weeks from birth. After sacrifice, animals were pinned down through the oral cavity and lightly stretched by the tail for nose-anus (body length) and anus-tail (tail length) measurements. To measure skeletal dimensions, limbs were subsequently removed and oriented in a consistent manner for X-ray photography and bone length measurement.

### EXAMPLE 5

### IGF-I measurements

Serum IGF-I levels in serum from orbital bleeds were determined using an ELA kit (rat IGF DSL-10-2900 Diagnostic Systems Laboratories, Webster, Tx) according to the manufacturer's instructions. IGF-I RNA expression was determined in RNase protections assays as previously described (23) using β-actin as an internal standard.

### EXAMPLE 6

### Generation and analysis of SOCS-2 knockout mice

To construct the SOCS-2 targeting vector, a 5' arm extending approximately 2.0 kb from the protein initiation ATG was generated by PCR using specific SOCS-2 oligonucleotides and genomic clone pgmSOCS-2 57-60-1-45 as template. This fragment was fused to the ATG of β-galactosidase *via* the *Bam*HI site in the plasmid vector pβgalpAloxneo. The 3' arm, a 3.7 kb *Eco*RI fragment from pgmSOCS-2 57-60-1-45 was blunted and ligated into the *Xho*I (blunted) site of pβgalphAloxneo that already contained the 5' arm. This targeting vector was linearized with *Not*I and electroporated into C57BL/6 embryonic stem cells. Transfected cells were selected in G418 and resistant clones picked and expanded. Clones in which the targeting vector has recombined with the endogenous SOCS-2 gene were identified by hybridising *Eco*RV-digested genomic DNA with a 1.8 kb *Eco*RI*-Eco*RV fragment from pgmSOCS-2 57-60-1-45. This probe (probe A, Figure 1), which is located 3' to the SOCS-2 sequences in the targeting vector, distinguished between the endogenous (greater than 14kb) and targeted (7.5 kb) SOCS-2 loci (Figure 1). Several targeted ES cells clones were identified, one of which was injected into Balb/c blastocysts to generate chimeric mice. Germline chimeras were mated with C57/BL/6 mice to produce SOCS-2^{+/-} mice which were interbred to yield SOCS-2-deficient animals. Southern blot analysis at weaning revealed that offspring of heterozygous parents included mice of each of the three expected genotypes in approximately Mendelian proportions (22:51:22 for SOCS-2^{+/+}:SOCS-2^{+/-}:SOCS-2^{-/-}). Northern blots of RNA extracted from a range of organs confirmed that SOCS-2 transcripts were absent in homozygous mutant mice. This result is consistent with the findings that the SOCS-2 gene had been functionally deleted.

SOCS-2^{-/-} mice appeared outwardly normal, however, male mice and perhaps to a lesser extend female mice developed to a significantly larger body weight than their normal littermates (Figure 2; males at 3 months of age: SOCS-2 -/- 41.2 ± 4.8, wild-type 29.3 ± 1.8; females: SOCS-2 -/- 25.3 ± 2.2, wild-type 21.9 ± 2.2 grams). In male mice the increased body weight was parallelled by increased size of several organs, in particular the pancreas, liver, lungs and heart (Figure 3). Upon removal of all organs the resulting means carcass weight also highlighted the increased size of SOCS-2^{-/-} mice (21.5 ± 1.5 g), compared to wild-type controls (13.1 ± 1.9 g). An extensive analysis has suggested that mice SOCS-2 -/- organs were histologically normal, with the exception of a marked thickening of the skin which develops as a result of increase collagen deposition. Both male and female SOCS-2 -/- have been found to be fertile. Adult SOCS-2^{+/-} males exhibited an intermediate body weight (-/-: 36.6 ± 1.0 g; +/-: 30.8 ± 1.3 g; +/+:27.1 ± 1.6 g, at 12 weeks of age, n = 5-20 mice per group). Increased growth was also significant but less dramatic in female SOCS-2^{-/-} mice. Adult SOCS-2^{-/-} females typically attained weights of wild-type male mice, but heterozygous SOCS-2 females were not significantly heavier that sex-matched wild-type littermates (-/-: 26.2 ± 1.5 g; +/-: 21.8 ± 0.7 g; +/+: 20.5 ± 1.4 g, at 12 weeks of age, n = 7-20 mice per group). Consistent with this interpretation, the femur, tibia, radius and humerus in SOCS-2^{-/-} mice were all significantly longer than in wild-type controls (Table 2). Body length in male SOCS-2-deficient mice was also greater, although tail length was normal (Table 2). The mean frequency of hepatic nuclei per 10 high power fields in SOCS-2^{-/-} liver sections was no greater than that in wild type mice (27.3 ± 4.4, n = 4 versus 27.7 ± 2.6, n = 4) and striated muscle cell width was normal in the thighs of SOCS-2-deficient animals. Thus, increased organ weights in these mice appear to have resulted from elevated cell numbers rather than increased cell size. A similar, but less pronounced trend was also observed when organ and carcass weights and body, tail and bone lengths were assessed in female SOCS-2^{-/-} mice (Table 2).

A thorough hematological survey has not identified any hematological abnormalities in SOCS-2 -/- mice (Table 3).

**TABLE 2 Body, tail and bone lengths in SOCS-2^{-/-} mice**

| **Length (mm)** | | | | | | |
|---|---|---|---|---|---|---|
| **Male** | | | | **Female** | | |
| | **SOCS-2^{+/+}** | **SOCS-2^{+/-}** | **SOCS-2^{+/+}** | **SOCS-2^{+/+}** | **SOCS-2^{+/-}** | **SOCS-2^{-/-}** |
| Body | 95.0 ± 1.9 | 98.8 ± 1.6* | 107 ± 0.9* | 90.1 ± 2.1 | 91.3 ± 1.2 | 100.3 ± 2.1* |
| Tail | 88.3 ± 1.5 | 87.7 ± 2.1 | 89.2 ± 1.5 | 84.8 ± 1.4 | 88.1 ± 1.6 | 88.9 ± 1.9* |
| Femur | 18.0 ± 0.2 | 18.7 ± 0.2* | 19.5 ± 0.2* | 17.3 ± 0.2 | 17.7 ± 0.2* | 18.6 ± 0.3* |
| Tibia | 20.0 ± 0.2 | 20.6 ± 0.2* | 20.9 ± 0.1* | 19.5 ± 0.2 | 19.9 ± 0.2* | 20.5 ± 0.3* |
| Radius | 12.0 ± 0.2 | 12.4 ± 0.2* | 12.7 ± 0.2* | 11.6 ± 0.2 | 11.7 ± 0.2 | 12.0 ± 0.2* |
| Humerus | 13.8 ± 0.2 | 14.4 ± 0.3* | 15.4 ± 0.2* | 13.2 ± 0.2 | 13.7 ± 0.3* | 14.6 ± 0.1* |

| | | | | | | |
|---|---|---|---|---|---|---|
| * p<0.005 in Student's t-test for comparison of sex-matched SOCS-2^{-/-} and SOCS-2^{+/-} data with SOCS-2^{+/+} mice (n = 6-12). | | | | | | |

**TABLE 3 Hematological profile of SOCS-2^{-/-} mice**

| | | **Genotype** | |
|---|---|---|---|
| | | **SOCS-2^{+/+}** | **SOCS-2^{-/-}** |
| **Peripheral Blood** | | | |
| Platelets (x10⁻⁶/ml) | | 855 ± 220 | 899 ± 183 |
| Haematocrit (%) | | 44 ± 2 | 44 ± 2 |
| White cell count (x10⁻⁶/ml) | | 7.3 ± 3.7 | 4.4 ± 1.2 |
| | Neutrophils (µl⁻¹) | 370 ± 360 | 350 ± 230 |
| | Lymphocytes (µl⁻¹) | 5070 ± 1130 | 5980 ± 2330 |
| | Monocytes (µl⁻¹) | 230 ± 70 | 310 ± 270 |
| | Eosinophils (µl⁻¹) | 50 ± 60 | 120 ± 140 |

| **Bone Marrow** | | | |
|---|---|---|---|
| | Blasts (%) | 3 ± 3 | 3 ± 1 |
| | Promyelocytes/Myelocytes (%) | 5 ± 2 | 7 ± 1 |
| | Metamyelocytes/Neutrophils (%) | 34 ± 6 | 29 ± 4 |
| | Lymphocytes (%) | 25 ± 7 | 27 ± 7 |
| | Monocytes (%) | 9 ± 1 | 7 ± 3 |
| | Eosinophils (%) | 3 ± 2 | 4 ± 2 |
| | Nucleated erythroid cells (5) | 21 ± 4 | 23 ± 7 |

| **Spleen** | | | |
|---|---|---|---|
| Weight | | 83 ± 14 | 119 ± 29 |
| | Blasts (%) | 2 ± 2 | 2 ± 2 |
| | Promyelocytes/Myelocytes (%) | 0 ± 0 | 0.5 ± 1 |
| | Metamyelocytes/Neutrophils (%) | 3 ± 1 | 2 ± 2 |
| | Lymphocytes (%) | 85 ± 5 | 86 ± 5 |
| | Monocytes (%) | 2 ± 2 | 3 ± 2 |
| | Eosinophils (%) | 1 ± 1 | 2 ± 1 |
| | Nucleated erythroid cells (%) | 7 ± 6 | 5 ± 2 |

| **Peritoneal cells** | | | |
|---|---|---|---|
| | Metamyelocytes/Neutrophils (%) | 1 ± 2 | 0 ± 0 |
| | Lymphocytes (%) | 39 ± 17 | 31 ± 19 |
| | Monocytes (%) | 66 ± 20 | 58 ± 17 |
| | Eosinophils (%) | 1 ± 1 | 0 ± 0 |
| | Mast cells | 2 ± 2 | 2 ± 0 |

### EXAMPLE 7

### Disruption of growth hormone signalling

The increased growth of mice is considered to be due to disruption in the pulsatile nature of growth hormone signalling in animals and in particular male animals.

To further analyze this, the phenomenon is studied and compared in animals homozygous and heterozygous for animals homozygous and heterozygous for the SOCS-2 gene. In these animals, levels of growth hormone and growth hormone controlled factors such as IGF-1 is determined.

The activation status of STAT5a and in particular STAT5b, which are major mediators of growth hormone and prolactin signalling, is determined in the heterozygous and homozygous mice. In addition, growth hormone, pulse-regulated, sexually dimorphic gene expression of genes such as MUP and CYP is also determined. Furthermore, the sensitivity of knock out mice to growth hormone signalling is further analyzed.

### EXAMPLE 8

### Effects of SOCS-2

To more directly examine the effects of SOCS-2 on the growth hormone system, the inventors initiated crosses of the SOCS-2 ^{-/-} mice with STAT5b^{-/-} and *little* mice. STAT5b is required for the sexually dimorphic effects of growth hormone. Consequently, male mice lacking SOCS-5b grow no larger than female mice, which are themselves normal size. A range of outcomes to this experiment is envisaged. If SOCS-2 is required to regulate growth hormone (GH) signalling, mice lacking both SOCS-2 and STAT5b are expected to reproduce a STAT-5b-deficient phenotype as GH signalling would not properly operate in the absence of this key signalling molecule making the presence or absence of SOCS-2 irrelevant. If SOCS-2 acts independently of GH signalling, an intermediate phenotype might ensue. Conversely, given that *little* mice are not entirely GH-deficient, if SOCS-2 acts to regulate GH signalling, a *little* mouse that is also deficient in SOCS-2 might exhibit amelioration of dwarfism.

### EXAMPLE 9

### Effects of STAT5b

If SOCS-2 acts on the GH or IGF-I signalling pathways, a strong prediction would be that the SOCS-2-deficient cells or the SOCS-2^{-/-} mice themselves would be significantly more sensitive than their wild-type counterparts to the effects of these cytokines. The deregulating of GH signalling might result in heightened activation of STAT5b, the key STAT involved in sexually dimorphic growth in mice. This is investigated in the livers of unmanipulated male SOCS-2^{-/-} mice, together with those from unmanipulated wild-type mice and wild-type mice after injection with a maximally stimulating dose of GH. The levels of expression of downstream markers of pulse-regulated GH signalling, including MUP, and cytochrome P450-catalysed testosterone hydroxylase, are determined in Northern blot analysis of liver RNA and/or Western blot analysis of protein extracts. Differences in GH sensitivity is also investigated by comparing the cytokine concentration required to stimulate phosphorylation of STAT5b in SOCS-2^{-/-} and normal mice in dose response studies using preparations of primary hepatocytes and/or fibroblasts.

The size parameters of SOCS-2/STAT5b are shown in Table 4. The data in the table show that male SOCS-2^{-/-} STAT5b^{-/-} mice have a growth phenotype intermediate to the large SOCS-2^{-/-} STAT5b^{+/+} and small SOCS-2^{+/+} STAT5b^{-/-} mice. In female mice, SOCS-2^{-/-} STAT5b^{-/-} also appear to be intermediate in this regard compared with mice lacking SOCS-2 or STAT5b singly. This result implies that the excessive growth in SOCS-2^{-/-} mice is dependent, at least in part, on STAT5b. Also, although not proof, this is further evidence consistent with abnormal growth hormone signalling in SOCS-2^{-/-} mice.

**TABLE 4 Size parameters for SOCS-2/STAT5b double knock-out mice**

| | **Genotype** | | | |
|---|---|---|---|---|
| | **SOCS-2^{+/+}/STAT5b^{+/+}** | **SOCS-2^{-/-}/STAT5b^{+/+}** | **SOCS-2^{+/+} STAT5b^{+-/-}** | **SOCS-2^{-/-} STAT5b^{-/-}** |
| **Male mice** | | | | |
| Body weight (g) | 28.4 ± 2.8 | 38.9 ± 2.0 | 19.5 ± 0.1 | 25.1 ± 1.0 |
| Carcass weight (g) | 7.8 ± 1.0 | 11.3 ± 1.0 | 5.2 ± 0.2 | 5.9 ± 0.4 |
| Body length (mm) | 99.9 ± 2.0 | 112.5 ± 3.6 | 88.0 ± 4.2 | 94.5 ± 2.1 |

| **Female mice** | | | | |
|---|---|---|---|---|
| Body weight (g) | 22.2 ± 2.6 | 28.3 ± 3.3 | 21.8 ± 2.2 | 24.4 ± 3.0 |
| Carcass weight (g) | 5.7 ± 0.7 | 8.2 ± 1.2 | 5.6 ± 0.4 | 6.3 ± 0.8 |
| Body length (mm) | 92.4 ± 4.4 | 106 ± 3.2 | 92.0 ± 0.8 | 95.4 ± 4.2 |

| | | | | |
|---|---|---|---|---|
| mean ± std dev of data from 2-8 mice per data point | | | | |

### EXAMPLE 10

### IGF signalling cascade

The possibility that SOCS-2 acts directly in the IGF-I signalling cascade is also being investigated. To achieve this, the phosphorylation status of the IGF-I receptor and key downstream signalling molecules in cells or tissues of SOCS-2^{-/-} mice is investigated. Primary embryo fibroblast cultures are established from SOCS-2^{-/-} and normal control mice. Initially, the extent and duration of phosphorylation of the IGF-I receptor, as well as the downstream effectors IRS and shc is measured following stimulation of these cells, or in primary hepatocyte preparations, with a maximally-stimulating dose of IGF-I. Differences in IGF-1 sensitivity are also investigated by comparing the minimal cytokine concentration required to stimulate receptor and substrate phosphorylation in SOCS-2^{-/-} and normal fibroblasts in dose response studies.

### EXAMPLE 11

### Constitutive SOCS-2 expression

Transgenic mice are generated which constitutively express SOCS-2 ubiquitously. The murine cDNA encoding SOCS-2 is linked to human ubiquitin C promoter, which drives high-level transgene expression in most mouse tissues. If the transgenic mice are small, and show evidence of resistance to GH or IGF-I, this provides strong evidence consistent with the hypothesis that SOCS-2 acts within the GH/IGF-I axis.

### EXAMPLE 12

### SOCS-2 regulation of major urinary protein (MUP) and insulin-like growth factor (IGF-1)

The inventors suspected that asspects of GH and/or IGF-I signalling might be deregulated in SOCS-2^{-/-} mice. To directly examine the GH/IGF-I pathway, the inventors first investigated levels of major urinary protein (MUP), a GH pulse-dependent product that is down-regulated in GH-overexpressing transgenic mice, where the usual pulsatile pattern of GH signalling is disrupted (21). To analyze MUP levels, 6-7 week old mice were made to urinate before samples were collected 3 and 5.5 hours later. Samples were pooled and centrifuged (13,000 rpm x 3 min) before 0.5 µl of supernatant was electorphoresed in 12% w/v SDS-polyacrylamide gels and stained with Coomassie blue. Consistent with deregulated GH signalling, less MUP was observed in samples of urine from each of 6 male and 5 female SOCS-2^{-/-} mice compared with a similar number of sex-matched wild-type samples (Figure 4a). Production of IGF-I is also stimulated by GH and, consistent with deregulated GH action, RNase protection assays revealed increased IGF-I production in several organs including the heart, lungs and spleen of SOCS-2^{-/-} mice (Figures 4b,c). Serum IGF-I levels in serum from orbital bleeds were determined using an EIA kit (rat IGF DSL-10-2900 Diagnostic Systems Laboratories, Webster, Tx) according to the manufacturer's instructions. IGF-I RNA expression was determined in RNase protections assays as previously described (23) using β-actin as an internal standard. However, excess production was not evident in the liver, bone, fat or muscle. No increase in serum IGF-I concentration was observed in SOCS-2^{-/-} mice (male -/-: 278 ± 74 ng/ml; +/+: 282 ± 45; female -/-: 310 ± 62; +/+: 298 ± 76; n = 6 mice per group), consistent with normal production in the liver, which is the major source of circulating IGF-I (22, 23).

Those skilled in the art will appreciate that the invention described herein is susceptible to variations and modifications other than those specifically described. It is to be understood that the invention includes all such variations and modifications. The invention also includes all of the steps, features, compositions and compounds referred to or indicated in this specification, individually or collectively, and any and all combinations of any two or more of said steps or features.

### BIBLIOGRAPHY

1. Nicola, N.A. Guidebook to cytokine and their receptors. Oxford University Press: Oxford, 1994.
2. Bazan, J.F. Immunology Today 11: 350-354, 1990.
3. Sprang et al, Curr. Opin. Structural Biol. 3: 815-827, 1993.
4. Hilton, D.J. Guidebook to cytokines and their receptors, 8-16: 1994.
5. Murakami et al. Proc. Natl. Acad. Sci. USA 88: 11349-11353, 1991.
6. Darnell et al, Science 264: 1415-1421, 1994.
7. Ihle, J.N. Nature 377: 591-594, 1995.
8. Ihle et al, Annual Review of Immunology 13: 369-398, 1995.
9. Sato et al, Embo Journal 12: 4181-4189; 1993.
10. Culter et al, Journal of Biological Chemistry 268: 21463-21465, 1993.
11. David et al, Journal of Biological Chemistry 271: 9185-9188, 1996.
12. Shual et al, Nature 366: 580-583, 1993.
13. David et al, Science 269: 1721-1723, 1995.
14. Gupta et al, Embo Journal 15: 1075-1084, 1996.
15. Wen et al, Cell 82: 241-250, 1995.
16. Altschul et al, Nucl. Acids Res. 25:3389. 1997.
17. Ausubel et al. "Current Protocols in Molecular Biology" John Wiley & Sons Inc, 1994-1998, Chapter 15.
18. Marmur and Doty, J. Mol. Biol. 5: 109, 1962
19. Bonner and Laskey, Eur. J. Biochem. 46: 83, 1974
20. Dey et al, J. Biol. Chem. 273:24095-24101, 1998
21. Norstedt et al, Cell 36:805-812, 1984
22. Yakar et al, Proc Natl Acad Sci USA 96:7324-7329, 1999
23. Sjogren et al, Proc Natl Acad Sci USA 96:7088-7092, 1999
24. Starr et al, Proc Natl Acad Sci USA 95:14395-14399, 1998
25. Alexander et al, EMBO J. 14:5569-5578, 1995
26. Alexander et al, Blood 87:2162-2170, 1996

## Claims

1. A genetically modified non-human animal exhibiting increased or decreased levels of a SOCS protein or a derivative or homologue thereof wherein the SOCS protein, in a genetically unaltered animal, comprises a protein:molecule interacting portion, N-terminal of a SOCS box, which SOCS box comprises the amino acid sequence:
X₁ X₂ X₃ X₄ X₅ X₆ X₇ X₈ X₉ X₁₀ X₁₁ X₁₂ X₁₃ X₁₄ X₁₅ X₁₆ [Xᵢ]ₙ X₁₇ X₁₈ X₁₉ X₂₀ X₂₁ X₂₂ X₂₃ [Xⱼ]ₙ X₂₄ X₂₅ X₂₆ X₂₇ X₂₈
wherein:
X₁ is L, I, V, M, A or P;
X₂ is any amino acid residue;
X₃ is P, T or S;
X₄ is L, I, V, M, A or P;
X₅ is any amino acid;
X₆ is any amino acid;
X₇ is L, I, V, M, A, F, Y or W;
X₈ is C, T or S;
X₉ is R, K or H;
X₁₀ is any amino acid;
X₁₁ is any amino acid;
X₁₂ is L, I, V, M, A or P;
X₁₃ is any amino acid;
X₁₄ is any amino acid;
X₁₅ is any amino acid;
X₁₆ is L, I, V, M, A, P, G, C, T or S;
[Xᵢ]ₙ is a sequence of n amino acids wherein n is from 1 to 50 amino acids and wherein the sequence Xᵢ may comprise the same or different amino acids selected from any amino acid residue;
X₁₇ is L, I, V, M, A or P;
X₁₈ is any amino acid;
X₁₉ is any amino acid;
X₂₀ is L, I, V, M, A or P;
X₂₁ is P;
X₂₂ is L, I, V, M, A, P or G;
X₂₃ is P or N;
[Xⱼ]ₙ is a sequence of n amino acids wherein n is from 0 to 50 amino acids and wherein the Xⱼ may comprise the same or different amino acids selected from any amino acid residue;
X₂₄ is L, I, V, M, A or P;
X₂₅ is any amino acid;
X₂₆ is any amino acid;
X₂₇ is Y or F;
X₂₈ is L, I, V, M, A or P.
and wherein the SOCS protein is SOCS-2 and comprises the amino acid sequence set forth in SEQ ID No. 2 or an amino acid sequence having at least 60% similarity thereto.

2. The genetically modified animal of claim 1 wherein the SOCS protein is encoded by the nucleotide sequence set forth in SEQ ID No. 1 or a nucleotide sequence having at least 60% similarity thereto or a nucleotide sequence capable of hybridizing to SEQ ID No. 1 or its complementary form under low stringency conditions at 42°C.

3. The genetically modified animal of claim 2 wherein the genetic modification results in a deletion of one or more nucleotides from a genomic sequence corresponding to SEQ ID No. 1 which substantially prevents, reduces or down-regulates expression of a genomic sequence corresponding to SEQ ID No. 1.

4. The genetically modified animal of any one of claims 1 to 3 wherein the animal is a mouse, rat, guinea-pig, rabbit or livestock animal.

5. The genetically modified animal of claim 4 wherein the animal is a mouse.

6. A method for screening for modulators of hormone signalling in an animal, said method comprising administering potential modulators to the genetically modified animal of claim 1 and identifying said modulators which alter hormone signalling.

7. The method of claim 6 wherein the genetically modified animal is a mouse.

8. Use of the genetically modified animal of claim 1 in the screening of a therapeutic molecule which modulates hormone signalling.

9. The method or use of any one of claims 6 to 8 wherein the hormone is growth hormone.

10. A non-medical method for modulating hormone signalling in a livestock animal or laboratory test animal, said method comprising up-regulating or down-regulating expression of a genetic sequence encoding a SOCS-2 protein or its derivative or homologue as defined in claim 1 or increasing or decreasing the activity of a SOCS-2 protein or its derivative or homologue as defined in claim 1 in said animal.

11. A non-medical method for controlling hormone signalling in a livestock animal or laboratory test animal, said method comprising up-regulating or down-regulating expression of a genetic sequence encoding a SOCS-2 protein or its derivative or homologue as defined in claim 1 or increasing or decreasing the activity of a SOCS-2 protein or its derivative or homologue as defined in claim 1 in said animal.

12. The method of claim 11 or 12 wherein the genetic sequence comprises the nucleotide sequence set forth in SEQ ID No. 1 or a nucleotide sequence having at least 60% similarity thereto or a nucleotide sequence capable of hybridizing to SEQ ID No. 1 or its complementary form under low stringency conditions at 42°C.

13. The method of any one of claim 10 to 12 wherein the hormone is growth hormone.

14. The method of any one of claims 10 to 12 wherein the hormone is insulin-like growth factor 1 (IGF-1) or prolactin.

15. A non-medical method for controlling growth hormone signalling in a livestock animal or laboratory test animal, said method comprising administering to said animal a control-effective amount of a SOCS-2 protein or functional part or homologue or analogue thereof as defined in claim 1 or antisense RNA or DNA, ribozymes or sense molecules that manipulate expression of SOCS-2 genes or translation of SOCS-2 mRNA, wherein said SOCS-2 gene and said SOCS-2 mRNA encode a SOCS-2 protein or derivative or homologue thereof as defined in claim 1, for a time and under conditions sufficient to modulate growth hormone signalling.

16. Use of a SOCS-2 protein or a derivative, homologue or analogue thereof as defined in claim 1 in the manufacture of a medicament for controlling hormone signalling in an animal.

17. Use of antisense RNA or DNA, ribozymes or sense molecules that manipulate expression of SOCS-2 genes or translation of SOCS-2 mRNA in the manufacture of a medicament for controlling hormone signalling in an animal, wherein said SOCS-2 gene and said SOCS-2 mRNA encodes a SOCS-2 protein or derivative or homologue thereof as defined in claim 1.

18. The use as claimed in claims 16 or 17 wherein expression of a specific sequence as defined in any one of claims 10 to 12 is up-regulated or down-regulated.

19. The use as claimed in any one of claims 16 to 18 wherein the hormone is growth hormone, preferably insulin-like growth factor 1 (IGF-1) or prolactin.

20. A use as claimed in any one of claims 16 to 19 for increasing strength in elderly people, obesity control treatment of catabolic diseases, treatment of chronic inflammatory disease, treatment and/or prophylaxis of osteoporosis, treatment of cardiomyopathy and in the treatment of complicated fracture.

21. The use as claimed in any one of claims 16 to 20 wherein the animal is a human, primate, livestock animal, laboratory test animal, companion animal or captive wild animal.

22. The use as claimed in claim 21 wherein said animal is a mouse, rat, guinea-pig or rabbit.

## Patentansprüche

1. Genetisch modifiziertes nichthumanes Tier, das erhöhte oder gesenkte Mengen eines SOCS-Proteins oder eines Derivats oder Homologen davon aufweist, wobei das SOCS-Protein in einem genetisch unveränderten Tier einen Protein-Molekül-Wechselwirkungsteil in N-terminaler Richtung von einer SOCS-Box umfasst, wobei die SOCS-Box die folgende Aminosäuresequenz umfasst:
X₁ X₂ X₃ X₄ X₅ X₆ X₇ X₈ X₉ X₁₀ X₁₁ X₁₂ X₁₃ X₁₄ X₁₅ X₁₆ [Xᵢ]ₙ X₁₇ X₁₈ X₁₉ X₂₀ X₂₁ X₂₂ X₂₃ [Xⱼ]ₙ X₂₄ X₂₅ X₂₆ X₂₇ X₂₈
wobei:
X₁ L, I, V, M, A oder P ist;
X₂ irgendein Aminosäurerest ist;
X₃ P, T oder S ist;
X₄ L, I, V, M, A oder P ist;
X₅ irgendein Aminosäurerest ist;
X₆ irgendein Aminosäurerest ist;
X₇ L, I, V, M, A, F, Y oder W ist;
X₈ C, T oder S ist;
X₉ R, K oder H ist;
X₁₀ irgendein Aminosäurerest ist;
X₁₁ irgendein Aminosäurerest ist;
X₁₂ L, I, V, M, A oder P ist;
X₁₃ irgendein Aminosäurerest ist;
X₁₄ irgendein Aminosäurerest ist;
X₁₅ irgendein Aminosäurerest ist;
X₁₆ L, I, V, M, A, P, G, C, T oder S ist;
[Xᵢ]ₙ eine Sequenz von n Aminosäuren ist, wobei n 1 bis 50 beträgt und wobei die Sequenz Xᵢ gleiche oder verschiedene Aminosäuren umfassen kann, die aus irgendeinem Aminosäurerest ausgewählt sind;
X₁₇ L, I, V, M, A oder P ist;
X₁₈ irgendein Aminosäurerest ist;
X₁₉ irgendein Aminosäurerest ist;
X₂₀ L, I, V, M, A oder P ist;
X₂₁ P ist;
X₂₂ L, I, V, M, A, P oder G ist;
X₂₃ P oder N ist;
[Xⱼ]ₙ eine Sequenz von n Aminosäuren ist, wobei n 0 bis 50 beträgt und wobei die Sequenz Xⱼ gleiche oder verschiedene Aminosäuren umfassen kann, die aus irgendeinem Aminosäurerest ausgewählt sind;
X₂₄ L, I, V, M, A oder P ist;
X₂₅ irgendein Aminosäurerest ist;
X₂₆ irgendein Aminosäurerest ist;
X₂₇ Y oder F ist;
X₂₈ L, I, V, M, A oder P ist;
und wobei es sich bei dem SOCS-Protein um SOCS-2 handelt, das die in SEQ ID Nr. 2 gezeigte Aminosäuresequenz oder eine Aminosäuresequenz, die wenigstens 60% Ähnlichkeit mit dieser aufweist, umfasst.

2. Genetisch modifiziertes Tier gemäß Anspruch 1, wobei das SOCS-Protein von der in SEQ ID Nr. 1 gezeigten Nucleotidsequenz oder einer Nucleotidsequenz, die wenigstens 60% Ähnlichkeit mit dieser aufweist, oder einer Nucleotidsequenz, die unter Bedingungen niedriger Stringenz bei 42 °C mit SEQ ID Nr. 1 oder deren komplementärer Form hybridisieren kann, codiert ist.

3. Genetisch modifiziertes Tier gemäß Anspruch 2, wobei die genetische Modifikation zu einer Deletion von einem oder mehreren Nucleotiden aus einer genomischen Sequenz, die SEQ ID Nr. 1 entspricht, führt, welche die Expression einer genomischen Sequenz, die SEQ ID Nr. 1 entspricht, im Wesentlichen verhindert, reduziert oder herunterreguliert.

4. Genetisch modifiziertes Tier gemäß einem der Ansprüche 1 bis 3, wobei das Tier eine Maus, Ratte, ein Meerschweinchen, Kaninchen oder Nutztier ist.

5. Genetisch modifiziertes Tier gemäß Anspruch 4, wobei das Tier eine Maus ist.

6. Verfahren zur Suche (Screening) nach Modulatoren der Hormonsignalübermittlung bei einem Tier, wobei das Verfahren das Verabreichen von potentiellen Modulatoren an das genetisch modifizierte Tier gemäß Anspruch 1 und das Identifizieren der Modulatoren, die die Hormonsignalübermittlung verändern, umfasst.

7. Verfahren gemäß Anspruch 6, wobei das genetisch modifizierte Tier eine Maus ist.

8. Verwendung des genetisch modifizierten Tiers gemäß Anspruch 1 bei der Suche nach einem therapeutischen Molekül, das die Hormonsignalübermittlung moduliert.

9. Verfahren oder Verwendung gemäß einem der Ansprüche 6 bis 8, wobei das Hormon ein Wachstumshormon ist.

10. Nichtmedizinisches Verfahren zum Modulieren der Hormonsignalübermittlung bei einem Nutztier oder Laborversuchstier, wobei das Verfahren das Hochregulieren oder Herunterregulieren der Expression einer genetischen Sequenz, die ein SOC-2-Protein oder ein Derivat oder Homologes davon, wie es in Anspruch 1 definiert ist, codiert, oder das Erhöhen oder Senken der Aktivität eines SOC-2-Proteins oder eines Derivats oder Homologen davon, wie es in Anspruch 1 definiert ist, bei dem Tier umfasst.

11. Nichtmedizinisches Verfahren zum Steuern der Hormonsignalübermittlung bei einem Nutztier oder Laborversuchstier, wobei das Verfahren das Hochregulieren oder Herunterregulieren der Expression einer genetischen Sequenz, die ein SOC-2-Protein oder ein Derivat oder Homologes davon, wie es in Anspruch 1 definiert ist, codiert, oder das Erhöhen oder Senken der Aktivität eines SOC-2-Proteins oder eines Derivats oder Homologen davon, wie es in Anspruch 1 definiert ist, bei dem Tier umfasst.

12. Verfahren gemäß Anspruch 11 oder 12, wobei die genetische Sequenz die in SEQ ID Nr. 1 gezeigte Nucleotidsequenz oder eine Nucleotidsequenz, die wenigstens 60% Ähnlichkeit mit dieser aufweist, oder eine Nucleotidsequenz, die unter Bedingungen niedriger Stringenz bei 42 °C mit SEQ ID Nr. 1 oder deren komplementärer Form hybridisieren kann, umfasst.

13. Verfahren gemäß einem der Ansprüche 10 bis 12, wobei das Hormon ein Wachstumshormon ist.

14. Verfahren gemäß einem der Ansprüche 10 bis 12, wobei es sich bei dem Hormon um insulinartigen Wachstumsfaktor 1 (IGF-1) oder Prolactin handelt.

15. Nichtmedizinisches Verfahren zum Steuern der Wachstumshormonsignalübermittlung bei einem Nutztier oder Laborversuchstier, wobei das Verfahren das Verabreichen einer steuerungswirksamen Menge eines SOCS-2-Proteins oder eines funktionellen Teils oder Homologen oder Analogen davon, wie es in Anspruch 1 definiert ist, oder einer Antisense-RNA oder -DNA, von Ribozymen oder Sense-Molekülen, die die Expression von SOCS-2-Genen oder die Translation von SOCS-2-mRNA manipulieren, wobei das SOCS-2-Gen und die SOCS-2-mRNA ein SOCS-2-Protein oder ein Derivat oder Homologes davon, wie es in Anspruch 1 definiert ist, codieren, an das Tier über einen Zeitraum und unter Bedingungen, die ausreichen, um die Wachstumshormonsignalübermittlung zu modulieren, umfasst.

16. Verwendung eines SOCS-2-Proteins oder eines Derivats, Homologen oder Analogen davon, wie es in Anspruch 1 definiert ist, bei der Herstellung eines Medikaments zur Steuerung der Hormonsignalübermittlung bei einem Tier.

17. Verwendung von Antisense-RNA oder -DNA, von Ribozymen oder Sense-Molekülen, die die Expression von SOCS-2-Genen oder die Translation von SOCS-2-mRNA manipulieren, bei der Herstellung eines Medikaments zur Steuerung der Hormonsignalübermittlung bei einem Tier, wobei das SOCS-2-Gen und die SOCS-2-mRNA ein SOCS-2-Protein oder ein Derivat oder Homologes davon, wie es in Anspruch 1 definiert ist, codieren.

18. Verwendung gemäß Anspruch 16 oder 17, wobei die Expression einer spezifischen Sequenz, wie sie in einem der Ansprüche 10 bis 12 definiert ist, hochreguliert oder herunterreguliert wird.

19. Verwendung gemäß einem der Ansprüche 16 bis 18, wobei das Hormon ein Wachstumshormon ist, vorzugsweise der insulinartige Wachstumsfaktor 1 (IGF-1) oder Prolactin.

20. Verwendung gemäß einem der Ansprüche 16 bis 19 zur Kräftigung von älteren Menschen, zur fettsuchtbekämpfenden Behandlung von katabolischen Krankheiten, zur Behandlung von chronischer Entzündungskrankheit, zur Behandlung und/oder Prophylaxe von Osteoporose, zur Behandlung von Kardiomyopathie und zur Behandlung einer komplizierten Fraktur.

21. Verwendung gemäß einem der Ansprüche 16 bis 20, wobei das Tier ein Mensch, Primat, Nutztier, Laborversuchstier, Haustier oder gefangenes Wildtier ist.

22. Verwendung gemäß Anspruch 21, wobei das Tier eine Maus, Ratte, ein Meerschweinchen oder Kaninchen ist.

## Revendications

1. Animal non humain modifié génétiquement présentant des niveaux accrus ou réduits d'une protéine SOCS ou d'un dérivé ou homologue de celle-ci, où la protéine SOCS, dans un animal non modifié génétiquement, comprend une partie d'interaction protéine : molécule, à l'extrémité N-terminale d'une boîte SOCS, laquelle boîte SOCS comprend la séquence d'acides aminés :
**X₁ X₂ X₃ X₄ X₅ X₆ X₇ X₈ X₉ X₁₀ X₁₁ X₁₂ X₁₃ X₁₄ X₁₅ X₁₆ [Xᵢ]ₙ X₁₇ X₁₈ X₁₉ X₂₀ X₂₁ X₂₂ X₂₃ [Xⱼ]ₙ X₂₄ X₂₅ X₂₆ X₂₇ X₂₈**
dans laquelle :
X₁ est L, I, V, M, A ou P;
X₂ est un quelconque résidu d'acide aminé ;
X₃ est P, T ou S;
X₄ est L, I, V, M, A ou P;
X₅ est un quelconque acide aminé
X₆ est un quelconque acide aminé ;
X₇ est L, I, V, M, A, F, Y ou W;
X₈ est C, T ou S ;
X₉ est R, K ou H ;
X₁₀ est un quelconque acide aminé ;
X₁₁ est un quelconque acide aminé ;
X₁₂ est L, I, V, M, A ou P;
X₁₃ est un quelconque acide aminé
X₁₄ est un quelconque acide aminé ;
X₁₅ est un quelconque acide aminé
X₁₆ est L, I, V, M, A, P, G, C, T ou S ;
[Xᵢ]ₙ est une séquence de n acides aminés dans laquelle n vaut de 1 à 50 acides aminés et dans laquelle la séquence Xᵢ peut comprendre des acides aminés identiques ou différents choisis parmi un quelconque résidu d'acide aminé ;
X₁₇ est L, I, V; M, A ou P ;
X₁₈ est un quelconque acide aminé ;
X₁₉ est un quelconque acide aminé ;
X₂₀ est L, I, V, M, A ou P ;
X₂₁ est P ;
X₂₂ est L, I, V, M, A, P ou G ;
X₂₃ est P ou N ;
[Xⱼ]ₙ est une séquence de n acides aminés dans laquelle n vaut de 1 à 50 acides aminés et dans laquelle la séquence Xⱼ peut comprendre des acides aminés identiques ou différents choisis parmi un quelconque résidu d'acide aminé ;
X₂₄ est L, I, V, M, A ou P ;
X₂₅ est un quelconque acide aminé ;
X₂₆ est un quelconque acide aminé ;
X₂₇ est Y ou F ;
X₂₈ est L, I, V, M, A ou P.
et dans laquelle la protéine SOCS est SOCS-2 et comprend la séquence d'acides aminés présentée dans SEQ ID n° 2 ou une séquence d'acides aminés ayant au moins 60 % de similarité avec celle-ci.

2. Animal modifié génétiquement selon la revendication 1, dans lequel la protéine SOCS est codée par la séquence nucléotidique présente dans SEQ ID n° 1 ou par une séquence nucléotidique ayant au moins 60 % de similarité avec celle-ci ou par une séquence nucléotidique capable de s'hybrider à SEQ ID n° 1 ou à sa forme complémentaire dans des conditions de faible stringence à 42°C.

3. Animal modifié génétiquement selon la revendication 2, dans lequel la modification génétique résulte dans une délétion d'un ou plusieurs nucléotides d'une séquence génomique correspondant à SEQ ID n° 1 qui prévient, régule négativement ou réduit sensiblement l'expression d'une séquence génomique correspondant à SEQ ID n° 1.

4. Animal modifié génétiquement selon l'une quelconque des revendications 1 à 3, l'animal étant une souris, un rat, un cobaye, un lapin ou un animal d'élevage.

5. Animal modifié génétiquement selon la revendication 4, l'animal étant une souris.

6. Procédé de criblage des modulateurs de la signalisation hormonale chez un animal, ledit procédé comprenant l'administration de modulateurs potentiels à l'animal modifié génétiquement selon la revendication 1 et l'identification desdits modulateurs qui modifient la signalisation hormonale.

7. Procédé selon la revendication 6, dans lequel l'animal modifié génétiquement est une souris.

8. Utilisation de l'animal modifié génétiquement selon la revendication 1 dans le criblage d'une molécule thérapeutique qui module la signalisation hormonale.

9. Procédé ou utilisation selon l'une quelconque des revendications 6 à 8, dans lequel l'hormone est une hormone de croissance.

10. Procédé non médical pour moduler la signalisation hormonale chez un animal d'élevage ou un animal de test de laboratoire, ledit procédé comprenant la régulation positive ou négative de l'expression d'une séquence génétique codant une protéine SOCS-2 ou de son dérivé ou homologue tel que défini dans la revendication 1, ou l'augmentation ou la diminution de l'activité d'une protéine SOCS-2 ou de son dérivé ou homologue tel que défini dans la revendication 1 chez ledit animal.

11. Procédé non médical pour contrôler la signalisation hormonale chez un animal d'élevage ou un animal de test de laboratoire, ledit procédé comprenant la régulation positive ou négative de l'expression d'une séquence génétique codant une protéine SOCS-2 ou de son dérivé ou homologue tel que défini dans la revendication 1 ou l'augmentation ou la diminution de l'activité d'une protéine SOCS-2 ou de son dérivé ou homologue tel que défini dans la revendication 1 chez ledit animal.

12. Procédé selon la revendication 11 ou 12, dans lequel la séquence génétique comprend la séquence nucléotidique présentée dans SEQ ID n° 1 ou une séquence nucléotidique ayant au moins 60 % de similarité avec celle-ci ou une séquence nucléotidique capable de s'hybrider à SEQ ID n° 1 ou à sa forme complémentaire dans des conditions de faible stringence à 42°C.

13. Procédé selon l'une quelconque des revenications 10 à 12, dans lequel l'hormone est une hormone de croissance.

14. Procédé selon l'une quelconque de revendications 10 à 12, dans lequel l'hormone est le facteur de croissance de type insuline 1 (IGF-1) ou la prolactine.

15. Procédé non médical pour contrôler la signalisation d'une hormone de croissance chez un animal d'élevage ou un animal de test de laboratoire, ledit procédé comprenant l'administration audit animal d'une quantité, efficace pour le contrôle, d'une protéine SOCS-2 ou d'une partie fonctionnelle ou d'un homologue ou d'un analogue de celle-ci, tel que défini dans la revendication 1, ou un ARN ou un ADN antisens, des ribozymes ou des molécules sens qui manipulent l'expression des gènes de SOCS-2 ou la traduction de l'ARNm de SOCS-2, dans lequel ledit gène de SOCS-2 et ledit ARNm de SOCS-2 codent une protéine SOCS-2 ou un dérivé ou homologue de celle-ci selon la revendication 1, pour une durée et des conditions suffisantes pour moduler la signalisation d'une hormone de croissance.

16. Utilisation d'une protéine SOCS-2 ou d'un dérivé ou d'un homologue ou d'un analogue de celle-ci selon la revendication 1 dans la fabrication d'un médicament destiné au contrôle de la signalisation hormonale chez un animal.

17. Utilisation d'un ARN ou ADN antisens, de ribozymes ou de molécules sens qui manipulent l'expression des gènes de SOCS-2 ou la traduction de l'ARNm de SOCS-2 dans la fabrication d'un médicament destiné à contrôler la signalisation hormonale chez un animal, dans laquelle ledit gène de SOCS-2 et ledit ARNm de SOCS-2 codent une protéine SOCS-2 ou un dérivé ou un homologue de celle-ci tel que défini dans la revendication 1.

18. Utilisation selon la revendication 16 ou 17, dans laquelle l'expression d'une séquence spécifique'telle que définie dans l'une quelconque des revendications 10 à 12 est régulée positivement ou négativement.

19. Utilisation selon l'une quelconque des revendications 16 à 18, dans laquelle l'hormone est une hormone de croissance, de préférence le facteur de croissance de type insuline 1 (IGF-1) ou la prolactine.

20. Utilisation selon l'une quelconque des revendications 16 à 19, pour fortifier les personnes âgées, le contrôle de l'obésité, le traitement des maladies métaboliques, le traitement d'une maladie inflammatoire chronique, le traitement et/ou la prophylaxie de l'ostéoporose, le traitement de la cardiomyopathie et dans le traitement d'une fracture compliquée.

21. Utilisation selon l'une quelconque des revendications 16 à 20, dans laquelle l'animal est un humain, un primate, un animal d'élevage, un animal de test de laboratoire, un animal de compagnie ou un animal sauvage captif.

22. Utilisation selon la revendication 21, dans laquelle ledit animal est une souris, un rat, un cobaye ou un lapin.
